# EUROPÄISCHE PATENTSCHRIFT

(11) **EP 3 706 626 B1**
(45) Veröffentlichungstag und Bekanntmachung des Hinweises auf die Patenterteilung: **03.01.2024**
(21) Anmeldenummer: 18804250.1
(22) Anmeldetag: 07.11.2018
(51) Int. Cl.: A61B 5/053

(54) **ÖSOPHAGUSELEKTRODENSONDE UND VORRICHTUNG ZUR KARDIOLOGISCHEN DIAGNOSE UND BEHANDLUNG**
OESOPHAGEAL ELECTRODE PROBE AND DEVICE FOR CARDIOLOGICAL DIAGNOSIS AND TREATMENT
SONDE À ÉLECTRODE SOPHAGIENNE ET DISPOSITIF POUR LE DIAGNOSTIC CARDIOLOGIQUE ET LE TRAITEMENT

(30) Priorität: 07.11.2017 DE 102017010318
(43) Veröffentlichungstag der Anmeldung: 16.09.2020
(73) Patentinhaber: Xenios AG, 74076 Heilbronn (DE)
(72) Erfinder: HEINKE, Matthias, 07407 Rudolstadt (DE); SCHALK, Marco, 67105 Schifferstadt (DE)
(74) Vertreter: Graf von Stosch, Andreas
(86) Internationale Anmeldenummer: PCT/EP2018/080382
(87) Internationale Veröffentlichungsnummer: WO 2019/091991

(56) Entgegenhaltungen:
- DE-A1-102004 001 626
- US-A1- 2003 097 167

## Beschreibung

Die Erfindung betrifft eine Ösophaguselektrodensonde bzw. einen Ösophaguskatheter zur Bioimpedanzmessung und zur Neurostimulation und eine Vorrichtung zur transösophagealen kardiologischen Behandlung und/oder kardiologischen Diagnose, welche eine Ösophaguselektrodensonde umfasst. Ferner ist offenbart, aber nicht Teil der Erfindung, ein Verfahren zum Steuern oder Regeln einer Ablationseinrichtung zum Durchführen einer Herzablation (insbesondere einer Herzkatheterablation) und/oder einer Herz-, Kreislauf- und/oder Lungenunterstützungseinrichtung zur herzsynchronen Herz-, Kreislauf- und/oder Lungenunterstützung.

Insbesondere betrifft die Erfindung eine Vorrichtung und eine Ösophaguselektrodensonde zur gerichteten transösophagealen Herzstimulation, Elektrokardiographie, Kardioversion, Bioimpedanzmessung und/oder Neurostimulation für transösophageale elektrophysiologische Untersuchungen des Herzens zur Diagnostik und Therapie von Herzrhythmusstörungen, zur Neurostimulation durch transösophageale Stimulation der aufsteigenden Nervenbahnen des Rückenmarks zur Unterdrückung der Nervenfortleitung für die Unterdrückung der Schmerzwahrnehmung im Gehirn und für neurologische elektrophysiologische Untersuchungen, für Messungen des Herzzeitvolumens, der Bioimpedanzen in unterschiedlichen Messabschnitten des Herzens und des Ösophagus, sowie anderer hämodynamischer, elektrokardiographischer und impedanzkardiographischer Parameter im Rahmen der temporären transösophagealen Vorhof- und/oder Ventrikelstimulation, Vorhof- und/oder Ventrikelelektrokardiographie, Bioimpedanzmessung und zur Optimierung der Energieabgabe von Wärme- und Kälteenergie sowie Laserenergie im Rahmen der Katheterablation bei tachykarder Herzrhythmusstörungen zur Vermeidung ösophagealer Verletzungen. Ferner betrifft die Erfindung eine Ösophaguselektrodensonde und eine Herz-, Kreislauf- und/oder Lungenunterstützungseinrichtung zur physiologischen herzsynchronen Herz-, Kreislauf- und/oder Lungenunterstützung, z.B. zur Behandlung von Hochrisikopatienten in der interventionellen Kardiologie und zur Behandlung des kardiogenen Schocks.

Im Stand der Technik sind verschiedene Verfahren und Ösophaguselektrodensonden zur transösophagealen Stimulation und Elektrokardiographie des Herzens zur Diagnostik und Therapie von Herzrhythmusstörungen und die transösophageale Impedanzkardiographie zur Messung des Herzzeitvolumens bekannt (DD 247608A1, DD 210207A1, DD 149610A1, DD 133400A1, DE 102004001626A1, DE 3327561A1, US 2003/0097167A1, US 2002/0198583A1, US 4836214, WO 81/03428, US 4574807, US 5056531, US 5967977, US 6006138, US 5431696, US 4304239, US 5191885, US5571150, US 5370679, US 5431696, D.J. McEneaney: An Esothoracic Electrode for Electrophysiological Studies: Journal of Electrophysiology Vol. 35 Supplement 2002, 151-157, H.R. Andersen, et al.: Trans-Esophageal Pacing, PACE 6, 1983. 674-679). Ebenfalls sind aus dem Stand der Technik Verfahren und Vorrichtungen zur Ablation zur Behandlung von Herzrhythmusstörungen, wie zum Beispiel Vorhofflimmern, bekannt. Zur Behandlung von Vorhofflimmern durch Isolation der Pulmonalvenen im linken Vorhof kann zum Beispiel eine Hochfrequenz-, Kryo-, Ultraschall- oder Laserablation eingesetzt werden.

Bei einer Ablation bzw. Katheterablation kann es jedoch zu lebensbedrohlichen Verletzungen des Ösophagus kommen. Um diese zu reduzieren oder zu vermeiden wird in einigen Kliniken die Katheterablation mittels ösophagealer Temperaturmessung überwacht. Eine beispielhafte Ösophagussonde zur Temperaturüberwachung ist die Ösophagussonde der Firma BISPING Medizintechnik GmbH, welche eine Temperaturüberwachung von -20° bis +65° ermöglicht und durch 12 Temperatursensoren den kompletten linken Vorhof abdeckt. Die kontinuierliche Temperaturüberwachung ist jedoch häufig unzureichend, um eine hohe Sicherheit der Ablation bzw. Katheterablation zu gewährleisten, da bei einem detektierten relevanten Anstieg der Temperatur bereits Gewebeverletzungen vorliegen.

Ferner verursachen kardiologische Behandlungen, wie z.B. eine transösophageale linksatriale und/oder linksventrikuläre Stimulation im Rahmen einer temporären kardialen Resynchronizationstherapie, zum Teil erhebliche Schmerzen bei den behandelten Patienten.

Es ist daher eine Aufgabe der Erfindung, eine verbesserte Ösophaguselektrodensonde und eine Vorrichtung bereitzustellen, die patientenschonendere und/oder weniger schmerzhafte kardiologische Behandlungen und/oder Messungen ermöglichen.

Diese Aufgabe wird durch eine Ösophaguselektrodensonde und eine Vorrichtung zur transösophagealen kardiologischen Behandlung und/oder Diagnose nach den unabhängigen Ansprüchen gelöst. Bevorzugte Ausführungsformen sind Gegenstand der abhängigen Ansprüche.

Es hat sich überraschenderweise herausgestellt, dass eine erhebliche Reduktion der durch eine kardiale Ablation bzw. Katheterablation hervorgerufenen Gewebeschädigungen durch eine Überwachung der Bioimpedanz mit Hilfe einer neuartigen Ösophaguselektrodensonde erreicht werden kann, bei der Elektroden zur Messung der Bioimpedanz sowohl auf der dem Herzen zugewandten Seite als auch auf der vom Herzen abgewandten Seite der Ösophaguselektrodensonde angeordnet sind. Mit Hilfe der vorgeschlagenen Ösophaguselektrodensonde ist es möglich, Gewebeveränderungen mittels einer transösophagealen Bioimpedanzmessung mit den dem Herzen zugewandten Elektroden zu überwachen und mit einer Bioimpedanzmessung mit den vom Herzen abgewandten Elektroden zu vergleichen, um Informationen abzuleiten und/oder darzustellen, auf Basis derer der weitere Fortgang der Ablation bzw. Katheterablation verändert werden kann. Gleichzeitig kann ein transösophageales hämodynamisches Monitoring durchgeführt werden.

Ferner hat sich überraschend herausgestellt, dass es durch eine transösophageale Neurosimulation auf der vom Herz abgewandten Seite einer Ösophaguselektrodensonde möglich ist, eine Schmerzreduktion bei einer transösophagealen Herzbehandlung zu erzielen, wie z.B. bei einer transösophagealen kardialen Stimulation oder einer transösophagealen Kardioversion zur Terminierung von Vorhofflimmern.

Ein erster Aspekt der Erfindung betrifft eine verbesserte Ösophaguselektrodensonde sowie eine verbesserte Vorrichtung zum transösophagealen Bioimpedanzmonitoring z.B. bei einer Ablation bzw. Katheterablation und/oder einer Herz-, Kreislauf- und/oder Lungenunterstützung. Die Ablation bzw. Katheterablation kann z.B. eine Hochfrequenz-, Kryo-, Ultraschall- oder Laserablation sein, z.B. eine Ablation zur Behandlung von Vorhofflimmern mittels Isolation der Pulmonalvenen im linken Vorhof.

Ein zweiter Aspekt der Erfindung (alternativ oder ergänzend zum ersten Aspekt) betrifft eine verbesserte Ösophaguselektrodensonde und eine Vorrichtung zur transösophagealen kardialen Stimulation, insbesondere zur transösophagealen linksatrialen und/oder linksventrikulären Stimulation im Rahmen der Diagnostik und Therapie bradykarder und tachykarder Herzrhythmusstörungen, wie zum Beispiel Initiierung und Terminierung von AV-Knoten-Reentry-Tachykardien (AVNRT), AV-Reentry-Tachykardien (AVRT) und Vorhofflattern, sowie zur transösophagealen linksventrikulären Stimulation im Rahmen einer temporären kardialen Resynchronisationstherapie bzw. einer antibradykarden temporären Herzstimulation, Die Ösophaguselektrodensonde weist mindestens eine Elektrode auf der vom Herzen abgewandten Seite der Ösophaguselektrodensonde zur Neurostimulation auf. Die vorgeschlagene transösophageale Neurostimulation ermöglicht eine Schmerzreduktion und eine Reduktion der Empfindungsschwelle der transösophagealen kardialen Stimulation.

Eine beispielhafte Ösophaguselektrodensonde gemäß einem ersten Aspekt der Erfindung umfasst eine Bioimpedanzmesseinrichtung zur Messung der Bioimpedanz von zumindest einem Teil des die Sonde umgebenden Gewebes. Die Bioimpedanzmesseinrichtung umfasst mindestens eine erste Elektrode und mindestens eine zweite Elektrode. Die mindestens eine erste Elektrode ist auf eine erste Seite der Sonde angeordnet. Die mindestens eine zweite Elektrode ist auf eine zweite Seite der Sonde angeordnet. Die erste Seite und die zweite Seite der Sonde sind in radialer Richtung der Sonde entgegengesetzt. Wenn die Ösophaguselektrodensonde in den Ösophagus des Patienten eingeführt ist, ist die erste Seite der Sonde dem Herzen zugewandt und die zweite Seite der Sonde vom Herzen abgewandt. Die Ösophaguselektrodensonde kann ferner eine Markierung aufweisen, die es ermöglicht, die dem Herz zugewandte und die vom Herzen abgewandte Seite zu identifizieren.

Die Ösophaguselektrodensonde umfasst ferner eine Neurostimulationeinrichtung Die Neurostimulation gemäß der Erfindung erfolgt als nichtkardiale Neurostimulation in Richtung Wirbelsäule, Zusätzlich kann eine kardiale Neurostimulation in Herzrichtung erfolgen. Die Neurostimulationeinrichtung umfasst mindestens eine Elektrode für eine transösophageale Neurostimulation des zumindest einen Teils des die Sonde umgebenden Gewebes mittels elektrischer Pulse mit einer Frequenz von 100 bpm bis 3000 bpm, vorzugsweise zwischen 1500 bpm und 2000 bpm, einer Stärke von ca. 5V bis 100V und einer Dauer von 3 Sekunden bis 10 Minuten, vorzugsweise zwischen 3 und 30 Sekunden. Weitere Parameter (nicht für die temporäre transösophageale Stimulation) einer invasiven Neurostimulation sind in Journal of Cardiovasc. Electrophysiol., Vol. 21, pp. 193-199, Februar 2010 beschrieben. Die mindestens eine Elektrode für die Neurostimulation kann auf der zweiten, vom Herzen abgewandten Seite der Ösophaguselektrodensonde angeordnet sein. Die zumindest eine Elektrode für die Neurostimulation ist insbesondere eingerichtet, eine Neurostimulation zur Schmerzreduktion bei transöphagealen Elektrostimulation zumindest eines Teils des die Ösophaguselektrodensonde umgebenden Gewebes zu erzielen. Es ist möglich, dass die Ösophaguselektrodensonde nur eine Neurostimulationeinrichtung aufweist, nicht jedoch eine Bioimpedanzmesseinrichtung.

Die Ösophaguselektrodensonde kann grundsätzlich wie eine herkömmliche Ösophaguselektrodensonde aufgebaut sein. Die Ösophaguselektrodensonde kann zum Beispiel einen länglichen, im Wesentlichen zylinderförmigen Sondenkörper, aufweisen. Der Sondenkörper kann zum Beispiel aus einem flexiblen Material sein. Der Sondenkörper weist ein proximales und ein distales Ende auf, wobei die Achse des Sondenkörpers im Wesentlichen mit der Einführrichtung der Ösophaguselektrodensonde in den Ösophagus des Patienten zusammenfällt. Anders als bei herkömmlichen Ösophaguselektrodensonden wird mit der vorliegenden Erfindung vorgeschlagen, dass Elektroden zur Bioimpedanzmessung sowohl auf der dem Herzen zugewandten als auch auf der vom Herzen abgewandten Seite der Sonde angeordnet sind. Ergänzend wird vorgeschlagen, dass Elektroden zur Neurostimulation auf der vom Herzen abgewandten Seite der Sonde angeordnet sind.

Die Elektroden können an den Sondenkörper angebracht sein. Vorzugsweise weist die Ösophaguselektrodensonde einen aufblasbaren Katheterballon aus einem geeigneten biokompatiblen elastischen Material auf, wobei der Katheterballon an den Sondenkörper mittels einer geeigneteren Befestigungsvorrichtung angebracht ist. Die Elektroden der Bioimpedanzmesseinrichtung und/oder der Neurostimulationeinrichtung können auf dem Katheterballon angeordnet sein. Im aufgeblasenen Zustand des Katheterballons treten die Elektroden vorzugsweise in Kontakt mit der Speiseröhre des Patienten.

Die einzelnen Elektroden können herkömmliche Elektroden zur Impedanzmessung und/oder Neurostimulation sein. Sie können eine im Wesentlichen halbzylindrische oder halbsphärische Form aufweisen, wobei die gekrümmte Fläche in Kontakt mit dem zu untersuchenden Gewebe tritt. Die Elektroden können aus einem biokompatiblen leitfähigen Material sein, wie beispielsweise Metall oder leitfähiger Kunststoff/Gummi.

Die Elektroden können ferner in Gruppen angeordnet werden, die jeweils eine oder mehrere Elektrodenreihen umfassen, wobei zwischen den einzelnen Elektroden jeweils ein konstanter oder unterschiedlicher Abstand sein kann. Die Elektroden können beliebig matrixförmig mit veränderbarem Elektroden-Myokard-Abstand angeordnet sein. Beispielsweise können die Elektroden in Reihen in Längsrichtung der Ösophaguselektrodensonde angeordnet sein.

Die Ösophaguselektrodensonde kann ferner weitere Einrichtungen zur Behandlung und/oder Untersuchung des Herzens und/oder anderer Körperorgane in der Umgebung des Ösophagus umfassen. Insbesondere kann die Ösophaguselektrodensonde ferner umfassen:
eine Stimulationseinrichtung mit mindestens einer Elektrode zur transösophagealen kardialen Stimulation, wobei die Elektrode auf der dem Herzen zugewandten Seite der Ösophaguselektrodensonde angeordnet ist; und/oder
eine Elektrographieeinrichtung mit mindestens einer Elektrode bzw. einem Sensor zur Elektrographiemessung; und/oder
eine Echokardiographieeinrichtung mit mindestens einem Ultraschallsensor zur Echokardiographiemessung; und/oder
eine Temperaturmesseinrichtung mit mindestens einem Temperatursensor zur Messung der Temperatur zumindest eines Teils des die Ösophaguselektrodensonde umgebenden Gewebes; und/oder
eine pH-Wert Messeinrichtung mit mindestens einem pH-Wert Sensor zur pH-Wert Messung zumindest eines Teils des die Ösophaguselektrodensonde umgebenden Gewebes.

Dies ermöglicht es, mehrere unterschiedliche Untersuchungen und/oder Behandlungen simultan oder zeitnah nacheinander mit einer Sonde durchzuführen, wie zum Beispiel Elektrokardiographie, Echokardiographie und kardiale Stimulation. Die aus den einzelnen Einrichtungen und/oder Sensoren gewonnen Messdaten können kombiniert oder gemeinsam ausgewertet werden, um zum Beispiel die Präzision einer Herzbehandlung (wie Herzablation bzw. Herzkatheterablation oder Herzstimulation) und/oder Herzuntersuchung zu verbessern. So können zum Beispiel Temperaturdaten und/oder Echographiedaten und/oder Elektrokardiographiedaten mit den Impedanzsignalen bzw. Impedanzmessdaten kombiniert werden, um die Präzision einer Herzablation bzw. Herzkatheterablation zu verbessern.

Ferner wird eine Vorrichtung zur transösophagealen kardiologischen Behandlung und/oder Diagnose vorgeschlagen, welche eine erfindungsgemäße Ösophaguselektrodensonde umfasst. Die Vorrichtung zur transösophagealen kardiologischen Behandlung und/oder Diagnose umfasst ferner eine Steuer- und/oder Auswerteinrichtung, wobei die Steuer- und/oder Auswerteinrichtung in Signalverbindung mit der Ösophaguselektrodensonde steht. Die Steuer- und/oder Auswerteinrichtung ist eingerichtet, Signale von dem zumindest einen Teil der Elektroden der Ösophaguselektrodensonde zu empfangen und auszuwerten und/oder Signale an zumindest einen Teil der Elektroden (wie zum Beispiel die Elektroden der Neurostimulationseinrichtung) zu senden.

Die Steuer- und/oder Auswerteinrichtung kann insbesondere in Signalverbindung mit der Bioimpedanzmesseinrichtung der Ösophaguselektrodensonde stehen und eingerichtet sein, die von den Elektroden der Bioimpedanzmesseinrichtung empfangenen Signale zu vergleichen und ein Kontrollsignal auf Basis des Vergleichs der empfangenen Signale zu generieren.

Alternativ oder zusätzlich kann die Steuer- und/oder Auswerteinrichtung in Signalverbindung mit einer Elektrographieeinrichtung der Ösophaguselektrodensonde stehen und eingerichtet sein, die von den Elektroden der Elektrographieeinrichtung empfangenen Signale zu vergleichen und ein Kontrollsignal auf Basis des Vergleichs der empfangenen Signale zu generieren.

Es ist auch möglich, das Kontrolsignal sowohl auf Basis der der empfangenen Signale der Bioimpedanzmesseinrichtung als auch der empfangenen Signale der Elektrographieeinrichtung zu generieren.

Das Kontrollsignal kann ein Statussignal sein, das den Status und/oder den Wert zumindest eines behandlungsrelevanten Parameters anzeigt. Die Behandlung kann zum Beispiel eine Herzablation bzw. Herzkatheterablation sein und das Kontrollsignal kann den Status bzw. den Wert zumindest eines Parameters der Herzablation bzw. Herzkatheterablation anzeigen, wie z.B. Energieabgabe, Temperatur, Fortschritt der Herzablation bzw. Herzkatheterablation, auftretende Gewebeschädigungen, etc. Das Statussignal kann ebenfalls den Status und/oder den Wert zumindest eines behandlungsrelevanten Parameters einer herzsynchronen Herz-, Kreislauf- und/oder Lungenunterstützung sein, wie z.B. Start, Ende, Umfang, etc. Das Statussignal kann auch ein optisches, akustisches oder haptisches Warnsignal sein, das anzeigt, dass sich zumindest ein behandlungsrelevanter Parameter außerhalb von einem zulässigen Wertebereich befindet oder größer/kleiner als ein vorbestimmter Schellenwert ist.

Das Kontrollsignal kann auch ein Steuersignal zum Steuern oder Regeln einer Vorrichtung zur Behandlung eines Patienten, wie zum Beispiel einer Herzablationseinrichtung bzw. Herzkatheterablationseinrichtung und/oder einer Herz-, Kreislauf- und/oder Lungenunterstützungseinrichtung zur physiologischen herzsynchronen Herz-, Kreislauf- und/oder Lungenunterstützung, sein. Das Steuersignal kann zumindest einen Parameter der Ablation bzw. Katheterablation und/oder der Herz-, Kreislauf- und/oder Lungenunterstützung steuern oder regeln, wie z.B. Intensität, Temperatur, Dauer oder räumliche Ausdehnung der Ablation; Volumen, Durchflussgeschwindigkeit, Sauerstoff- und/oder Kohlendioxidtransfer, Temperatur, Dauer, etc. bei einer Herz-, Kreislauf- und/oder Lungenunterstützungseinrichtung. Das Steuersignal kann insbesondere ein Signal sein, das die Ablation bzw. Katheterablation und/oder die Herz-, Kreislauf- und/oder Lungenunterstützung automatisch beendet oder startet oder die Ablationstemperatur und/oder den Unterstützungsumfang steuert oder regelt.

Vorzugsweise wird während einer Herzablation bzw. Herzkatheterablation und/oder einer Herz-, Kreislauf- und/oder Lungenunterstützung die Bioimpedanz kontinuierlich überwacht, wobei der Abstand zwischen zwei nacheinander folgenden diskreten Bioimpedanzmessungen vorzugsweise 5 Sekunden, besonders bevorzugt 1 Sekunde beträgt. Ferner ist eine Mittelung von zum Beispiel 3 bis 5 Herzaktionen möglich. Aus den einzelnen Bioimpedanzmessungen ergibt sich ein zeitlich variables Bioimpedanzsignal, z.B. in Form eines Impedanzkardiograms.

Die in einem bestimmten Zeitpunkt gemessene Bioimpedanz der ersten Elektrode kann mit der in diesem Zeitpunkt gemessenen Bioimpedanz der zweiten Elektrode verglichen werden. Anhand der Differenz der beiden Werte kann ein Signal bzw. Informationen abgeleitet werden, das bzw. die zumindest einen Parameter der Ablation bzw. Katheterablation (wie z.B. Energieabgabe von Wärme- oder Kälteenergie, Laserenergie, Ultraschallenergie, Temperatur, Dauer, räumliche Ausdehnung, Start, Ende, etc.) und/oder der Herz-, Kreislauf- und/oder Lungenunterstützung (wie z.B. Volumen, Durchflussgeschwindigkeit, Sauerstoff- und/oder Kohlendioxidtransfer, Temperatur, Dauer, Start, Ende, etc.) beeinflussen oder optimieren können. Zu hohe Energieabgabe, z.B. bei Vorhofflimmerablation, kann zur Schädigung des Ösophagus führen, die zu Vorhof-Ösophagus-Fisteln zwischen linkem Atrium und Ösophagus führen kann. Ziel der Optimierung kann z.B. die Erkennung und Verhinderung zu hoher Energieabgaben bei der Ablation bzw. Katheterablation sein.

Es ist ebenfalls möglich, anhand der einzelnen Bioimpedanzmessungen der jeweils mindestens einen ersten Elektrode und der mindestens einen zweiten Elektrode innerhalb eines bestimmten Zeitintervalls charakteristische Signalparameter zu ermitteln, die miteinander verglichen werden können und anhand derer ein Steuersignal generiert werden kann. Ein beispielhafter charakteristischer Signalparameter kann ein gewichteter oder ungewichteter Mittelwert der einzelnen Impedanzmessungen innerhalb eines bestimmten Zeitintervalls sein. Andere beispielhafte Parameter können die Form der zeitlich variablen Impedanzsignale, die Steigung bestimmter Signalabschnitte, der Abstand zwischen den Signalmaxima und/oder Signalminima, etc. sein. Neben dem Vergleich von Parametern im Zeitbereich können Parameter im spektralen Bereich (FFT, Spektro-Temporales Mapping, Wavelet Analyse, etc.) verglichen werden.

Bei mehreren ersten Elektroden, d.h. mehreren auf der ersten Seite der Ösophaguselektrodensonde angeordneten Elektroden kann ein erstes Bioimpedanzmessignal aus den einzelnen Messsignalen aller ersten Elektroden gebildet werden, zum Beispiel durch Bilden einer gewichteten oder ungewichteten Summe, eines gewichteten oder ungewichteten Mittelwerts, eines Medianwertes, etc. Entsprechendes gilt auch für den Fall, dass mehrere zweite Elektroden vorhanden sind: ein zweites Bioimpedanzmessignal kann aus den einzelnen Messsignalen aller zweiten Elektroden gebildet werden.

Gemäß einem bevorzugten Beispiel wird das Kontrollsignal (z.B. Warnsignal, Statussignal, Steuersignal) anhand der Differenz zwischen dem ersten Bioimpedanzmesssignal und dem zweiten Bioimpedanzmesssignals gebildet. Das Kontrollsignal kann auch anhand der Differenz zwischen Elektrokardiographiesignalen, z.B. der Differenz zwischen den Elektrokardiographiesignalen einer oder mehrerer herznaher und einer oder mehrerer herzfernerer Ösophaguselektroden, und aus der Kombination von Bioimpedanzsignalen und Elektrokardiographiesignalen gebildet werden. Weiterhin können diese transösophagealen Bioimpedanzsignale und/oder Elektrokardiographiesignale mit transthorakalen Bioimpedanzsignalen und/oder Elektrokardiographiesignalen und/oder intrakardialen Bioimpedanzsignalen und/oder Elektrokardiographiesignalen gebildet werden.

Das Kontrollsignal kann ein Signal zum Beenden einer Ablation bzw. Katheterablation und/oder einer Herz-, Kreislauf- und/oder Lungenunterstützung sein, wenn die Differenz zwischen dem ersten Bioimpedanzmesssignal und dem zweiten Bioimpedanzmesssignal gleich oder größer als ein vorbestimmter Schwellenwert ist. Der Schwellenwert ist vorzugsweise individuell vom Untersucher einstellbar und kann auch von der Art der Katheterablation und den Erfahrungen des Rhythmologen abhängen. Beispielsweise kann der Schwellenwert bei größer zehn Prozent liegen.

Es ist ebenfalls möglich, alternativ oder zusätzlich zeitliche und/oder räumliche und/oder spektrale Signalmuster zu vergleichen und anhand des Unterschieds in den Mustern ein Kontrollsignal zu generieren.

Die Vorrichtung kann ferner eine Ablationseinrichtung zur Ablation bzw. Katheterablation zumindest eines Teils des die Ösophaguselektrodensonde umgebenden Gewebes umfassen, z.B. eine Herzablationseinrichtung bzw, Herzkatheterablationseinrichtung. Die Ablationseinrichtung kann insbesondere eine Katheterablationseinrichtung zur Katheterablation von Herzrhythmusstörungen sein. Die Katheterablationseinrichtung kann eingerichtet sein, eine Hochfrequenz-, eine Kryo-, eine Ultraschall- oder eine Laserablation durchzuführen. Die Katheterablationseinrichtung kann z.B. ein Herzkatether sein oder einen Herzkatheter umfassen. Die Katheterablationseinrichtung kann eine der Sonde externe Vorrichtung sein oder in der Sonde selbst integriert sein. Weiterhin ist eine Kommunikation und/oder ein Vergleich der Signale zwischen der Ösophaguselektrodensonde und intrakardialen Ablationselektroden denkbar.

Die Vorrichtung kann ebenfalls eine Herz-, Kreislauf- und/oder Lungenunterstützungseinrichtung zur physiologischen herzsynchronen Herz-, Kreislauf- und/oder Lungenunterstützung umfassen, die in Signalverbindung mit der Ösophaguselektrodensonde steht. Die Herz-, Kreislauf- und/oder Lungenunterstützungseinrichtung kann z.B. zur Behandlung von Hochrisikopatienten in der interventionellen Kardiologie und zur Behandlung des kardiogenen Schocks eingesetzt werden. Die Vorrichtung kann ferner eine Anzeigeeinrichtung umfassen, die in Signalverbindung mit der Ösophaguselektrodensonde steht, und die eingerichtet ist, die von dem zumindest einen Teil der Elektroden der Ösophaguselektrodensonde empfangenen Signale, das Resultat einer Auswertung derer und/oder das Kontrollsignal anzuzeigen. Die Anzeigeneinrichtung kann ferner Signale anzeigen, die an zumindest einen Teil der Elektroden der Ösophaguselektrodensonde gesendet worden sind oder gesendet werden Die Anzeigeeinrichtung kann zum Beispiel in Signalverbindung mit der Bioimpedanzmesseinrichtung der Ösophaguselektrodensonde stehen und die Bioimpedanzmesssignale und/oder ein anderes daraus abgeleitetes Signal (wie z.B. deren Differenz) in geeigneter Form anzeigen.

Ferner ist es möglich, dass mehrere Elektroden durch elektrischen Kurzschluss mittels Adapter oder Schaltvorrichtung zu einer Elektrode für die unipolare Kardioversion oder zu zwei Elektroden für die unipolare oder bipolare Kardioversion zusammengeschlossen werden, wobei die einzelnen Elektroden aus leitfähigem Material, wie beispielsweise Metall oder leitfähigem Kunststoff/Gummi, bestehen.

Ein weiterer Aspekt der nicht Teil der Erfindung ist betrifft ein Verfahren zum Steuern oder Regeln einer Herzablationseinrichtung bzw. Herzkatheterablationseinrichtung (Ablationseinrichtung zum Durchführen einer Herzablation bzw. Herzkatheterablation) und/oder einer Herz-, Kreislauf- und/oder Lungenunterstützungseinrichtung. Das Verfahren umfasst:
Erfassen eines ersten Bioimpedanzmesssignals von mindestens einer ersten Elektrode, wobei die mindestens eine erste Elektrode auf einer dem Herzen zugewandten Seite einer Ösophaguselektrodensonde angeordnet ist;
Erfassen eines zweiten Bioimpedanzmesssignals von mindestens einer zweiten Elektrode, wobei die mindestens eine zweite Elektrode auf einer vom Herzen abgewandten Seite der Ösophaguselektrodensonde angeordnet ist;
Generieren eines Steuersignals zum Steuern oder Regeln der Herzablationseinrichtung bzw. Herzkatheterablationseinrichtung und/oder der Herz-, Kreislauf- und/oder Lungenunterstützungseinrichtung auf Basis eines Vergleichs des ersten Bioimpedanzmesssignals mit dem zweiten Bioimpedanzmesssignal.

Alternativ oder zusätzlich kann das Verfahren zum Steuern oder Regeln einer Herzablationseinrichtung bzw. Herzkatheterablationseinrichtung (Ablationseinrichtung zum Durchführen einer Herzablation bzw. Herzkatheterablation) und/oder einer Herz-, Kreislauf- und/oder Lungenunterstützungseinrichtung die folgenden Schritte umfassen:
Erfassen eines ersten Elektrokardiographiemesssignals von mindestens einer ersten Elektrode, wobei die mindestens eine erste Elektrode auf einer dem Herzen zugewandten Seite einer Ösophaguselektrodensonde angeordnet ist; und
Erfassen eines zweiten Elektrokardiographiemesssignals von mindestens einer zweiten Elektrode, wobei die mindestens eine zweite Elektrode auf einer vom Herzen abgewandten Seite der Ösophaguselektrodensonde angeordnet ist.

Ferner kann das Verfahren umfassen:
Generieren eines Steuersignals zum Steuern oder Regeln der Herzablationseinrichtung bzw. Herzkatheterablationseinrichtung und/oder der Herz-, Kreislauf- und/oder Lungenunterstützungseinrichtung auf Basis eines Vergleichs des ersten Elektrokardiographiemesssignals mit dem zweiten Elektrokardiographiemesssignal.

Die Ösophaguselektrodensonde kann eine Ösophaguselektrodensonde gemäß einem Aspekt der Erfindung sein. Das Verfahren kann dementsprechend ein Bereitstellen einer Ösophaguselektrodensonde gemäß einem Aspekt der Erfindung umfassen.

Das Steuersignal kann insbesondere das oben beschriebene Steuersignal sein. Insbesondere kann das Steuersignal ein Signal zum Beenden einer von der Herzablationseinrichtung bzw. Herzkatheterablationseinrichtung durchgeführten Ablation bzw. Katheterablation und/oder einer Herz-, Kreislauf- und/oder Lungenunterstützung sein, wenn die Differenz zwischen dem ersten Bioimpedanzmesssignal und dem zweiten Bioimpedanzmesssignal und/oder die Differenz zwischen dem ersten Elektrokardiographiemesssignal und dem zweiten Elektrokardiographiemesssignal gleich oder größer als ein vorbestimmter Schwellenwert ist.

Ein weiterer Aspekt der nicht Teil der Erfindung ist betrifft ein Verfahren zum Herstellen einer Ösophaguselektrodensonde, z.B. einer Ösophaguselektrodensonde gemäß einem Aspekt der Erfindung sein. Das Verfahren umfasst ein Herstellen der Ösophaguselektrodensonde mittels eines 3D Druckverfahrens. Das Verfahren kann ferner Bereitstelen von Daten für das 3D Druckverfahren umfassen. Die Daten können z.B. die Form, die Dimensionen, die Elektroden und deren Anordnung, die Materialien für die einzelnen Komponenten, und/oder andere notwendige Daten für das 3D Druckverfahren umfassen. Die Daten können z.B. in einer Datenbank oder auf einem anderen geeigneten Speichermedium gespeichert werden. Die Daten können in Form von 3-D CAD Daten oder in anderen geeigneten Formaten vorliegen. Die Daten können mit Hilfe eines Herzmodels erstellt und/oder getestet werden. Das Herzmodell kann z.B. anhand von durchschnittlichen oder patientenspezifischen Patienten- und/oder physiologischen Daten erstellt werden. Ein beispielhaftes Herzmodell wird nachfolgend in Detail beschrieben.

Die Erfindung soll nachstehend anhand von in der Zeichnung dargestellten Ausführungsbeispielen näher erläutert werden. Es zeigen:
**Fig. 1** eine beispielhafte Vorrichtung zum transösophagealen Bioimpedanzmonitoring und/oder zur kardialen Stimulation und/oder EKG und/oder kardialen Neurostimulation, insbesondere zur temporären transösophagealen Linksherzstimulation und/oder Linksherzelektrokardiographie und/oder Neurostimulation;
**Fig. 2** eine beispielhafte Vorrichtung zum transösophagealen Bioimpedanzmonitoring bei kardialer Resynchronisationstherapie oder zur Optimierung einer Herz-, Kreislauf- und Lungenunterstützungseinrichtung;
**Fig. 3** eine beispielhafte Vorrichtung zur transösophagealen Kardioversion von Vorhofflimmern und/oder transösophagealen Linksherzelektrokardiographie;
**Fig. 4** die Ergebnisse einer Kombination von temporärer transösophagealer hochfrequenter Vorhofstimulation und temporärer transösophagealer Neurostimulation mit Reduktion der Reizschwelle der transösophagealen Vorhofstimulation;
**Fig. 5** eine beispielhafte Ösophaguselektrodensonde zur temporären Linksherzstimulation mittels bipolarer transösophagealer linksatrialer und bipolarer linksventrikulärer Stimulation und temporäre transösophageale Neurostimulation;
**Fig. 6** eine beispielhafte Ösophaguselektrodensonde zur temporären transösophagealen linksventrikulären Stimulation mit reduziertem Elektroden-Myokard-Abstand und temporären transösophagealen Neurostimulation;
**Fig. 7** eine beispielhafte Ösophaguselektrodensonde zur temporären transösophagealen linksventrikulären Stimulation mit reduziertem Elektroden-Myokard-Abstand und temporären transösophagealen Neurostimulation mit reduziertem Elektroden-Rückenmark-Abstand;
**Fig. 8** bis **11** beispielhafte Ösophaguselektrodensonden mit einem aufblasbaren Katheterballon zur temporären transösophagealen linksatrialen und/oder linksventrikulären Stimulation, und/oder zur Elektrokardiographie, und/oder zum hämodynamischen Monitoring mit reduziertem Elektroden-Myokard-Abstand, und/oder zur temporären transösophagealen Neurostimulation mit reduziertem Elektroden-Rückenmark-Abstand;
**Fig. 12** bis **14** beispielhafte Ösophaguselektrodensonden zur temporären transösophagealen Stimulation, und/oder Elektrokardiographie, und/oder Bioimpedanzmessung, und/oder Katheterablation und/oder Kardioversion und/oder Herzstimulation und/oder kardiale Neurostimulation und/oder temporären transösophagealen Neurostimulation ohne Katheterballon (Fig. 12), mit einem nicht aufgeblasenem Katheterballon (Fig. 13) und mit einem aufgeblasenem Katheterballon (Fig. 14);
**Fig. 15** eine beispielhafte Ösophaguselektrodensonde zur Neurostimulation und/oder bipolaren DC AF Terminierung;
**Fig. 16** eine beispielhafte Ösophaguselektrodensonde zur Neurostimulation und/oder unipolaren DC AF Terminierung;
**Fig. 17** ein beispielhaftes 3D-CAD Herzmodell mit einer Ösophaguselektrodensonde;
**Fig. 18** eine beispielhafte kardiale Neurostimulation bei linksatrialer und linksventrikulärer Stimulation; und
**Fig. 19** eine beispielhafte kardiale Neurostimulation und transthorakale und transösophageale Elektrokardiographie bei Sinusrhythmus und Schenkelblockierung.

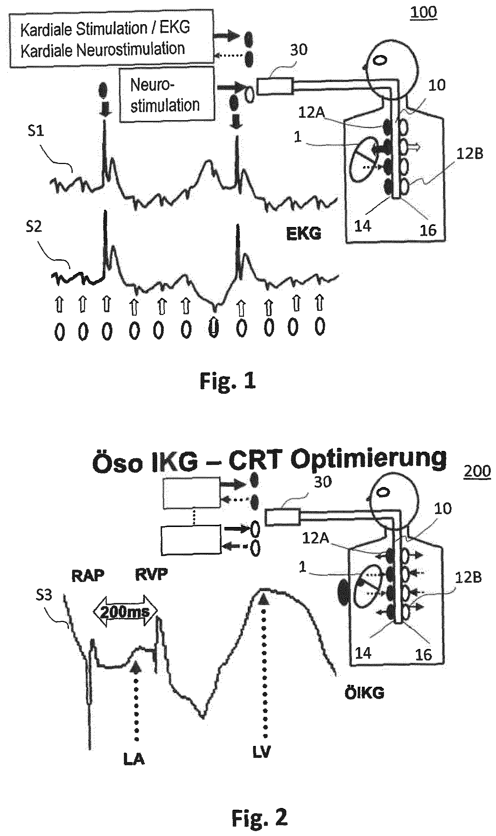

**Fig. 1** zeigt schematisch eine beispielhafte Vorrichtung zum transösophagealen Bioimpedanzmonitoring und/oder zu weiteren Messungen und Behandlungen, wie z.B. zur kardialen Stimulation, EKG und/oder kardialen Neurostimulation, insbesondere zur temporären transösophagealen Linksherzstimulation und/oder Linksherzelektrokardiographie und/oder Neurostimulation 100 (als Beispiel einer Vorrichtung zur transösophagealen kardiologischen Behandlung und/oder Diagnose). Die Vorrichtung 100 umfasst eine Ösophaguselektrodensonde 10 mit mehreren Elektroden 12A auf der dem Herzen 1 zugewandten (herznahen) Seite 14 und mit mehreren Elektroden 12B auf der vom Herzen abgewandten Seite 16 der Ösophaguselektrodensonde 10. In den Figuren sind die Elektroden 12A auf der herznahen Seite 14 der Ösophaguselektrodensonde 10 durch schwarz gefüllte Ellipsen oder Kreise und die Elektroden 12B auf der herzabgewandten Seite 16 der Ösophaguselektrodensonde 10 durch nicht gefüllte Ellipsen oder Kreise dargestellt. Die herznahe und die herzabgewandte Seite der Ösophaguselektrodensonde 10 können z.B. durch entsprechende Markierungen auf der Ösophaguselektrodensonde 10 markiert werden, die eine kontrollierte Platzierung der Sonde in Bezug auf das Herz 1 ermöglichen.

Die Elektroden 12A und 12B sind jeweils in Reihen in Längsrichtung bzw. entlang der Länge der Ösophaguselektrodensonde 10 angeordnet, wobei mindestens eine Reihe von Elektroden 12A auf der herznahen Seite 14 und mindestens eine Reihe von Elektroden 12B auf der herzabgewandten 16 Seite der Ösophaguselektrodensonde 10 angeordnet sind. Die Elektroden 12 umfassen Elektroden zur Bioimpedanzmessung, die sowohl auf der herznahen Seite 14 als auch auf der herzabgewandten Seite 16 der Ösophaguselektrodensonde angeordnet sind, Elektroden zur temporären transösophagealen Linksherzstimulation und/oder Linksherzelektrokardiographie und Elektroden zur Neurostimulation, die auf der herzabgewandten Seite 16 der Ösophaguselektrodensonde 10 angeordnet sind. Die Neurostimulation kann insbesondere für die Schmerzreduktion bei transösophagealer Elektrostimulation und/oder zur Reizschwellenreduktion bei transösophagealer linksventrikulärer und linksatrialer Stimulation eingesetzt werden und kann z.B. durch hochfrequente elektrische Signale mit einer Frequenz von 100 bpm bis 1200 bpm, vorzugsweise von 100 bpm bis 300 bpm, einer Stärke von ca. 5V bis 50V bei einer Impulsbreite von 2 bis 20 Millisekunden für eine Dauer von 2 Sekunden bis 30 Sekunden erfolgen.

Die Ösophaguselektrodensonde 10 kann auch zusätzliche Elektroden oder Sensoren umfassen, wie z.B. Elektroden zur transösophagealen linksventrikulären Kammerstimulation und/oder transösophagealen linksatrialen Vorhofstimulation auf der herznahen Seite 14 der Ösophaguselektrodensonde 10 oder Elektrokardiographieelektroden (EKG Elektroden) auf der herznahen Seite 14 der Ösophaguselektrodensonde 10 zur Linksherzelektrokardiographie.

Die einzelnen Elektroden 12 können herkömmliche Elektroden sein, die zumindest teilweise aus einem leitfähigen Material sind. Die Elektroden 12 können zum Beispiel eine im Wesentlichen halbsphärische oder halbzylindrische Form aufweisen, wobei die gewölbte Fläche in Kontakt mit der Speiseröhre des Patienten tritt.

Die Elektroden 12 sind über Signalleitungen mit einer Steuer- und Auswertevorrichtung 30 verbunden. Die Steuer- und Auswertevorrichtung 30 kann eine externe oder eine in der Ösophaguselektrodensonde 10 integrierte Vorrichtung sein. Bei der in Fig. 1 gezeigten Vorrichtung ist die Steuer und Auswerteeinrichtung 30 außerhalb der Ösophaguselektrodensonde 10 angeordnet.

Zwei oder mehrere der Elektroden 12 können zusammengeschaltet werden. Beispielsweise können zwei zusammengeschaltete und/oder angesteuerte Elektroden 12 als eine bipolare Vorhofelektrode zur transösophagealen Vorhofstimulation oder Vorhofwahrnehmung oder als eine bipolare Kammerelektrode zur transösophagealen Kammerstimulation oder Kammerwahrnehmung eingesetzt werden. Vier Elektroden 12 können zu einer unipolaren Elektrode zur unipolaren Kardioversion von Vorhofflattern oder Vorhofflimmern mit einer transthorakalen oder intrakardialen Gegenelektrode zusammengeschaltet werden. Durch Parallelbetrieb von je zwei distalen und zwei proximalen Elektroden ist zum Beispiel eine transösophageale bipolare Kardioversion möglich.

Die Elektroden 12 stehen in Signalverbindung mit einer Steuer- und/oder Auswerteinrichtung 30, welche die Signale von den Elektroden 12 (z.B. von den Elektroden zur Bioimpedanzmessung) auswertet und/oder Signale (z.B. Steuersignale) an die Elektroden 12 und gegebenenfalls weitere Einrichtungen sendet. Die von den Elektroden empfangenen Signale und/oder das Resultat der Auswertung derer können auf einer Anzeigeeinrichtung angezeigt werden. Die Steuer- und/oder Auswerteinrichtung 30 ist ferner eingerichtet, anhand der empfangenen Signale Auswertungssignale (wie z.B. die oben beschriebenen Warnsignale, Statussignale und/oder Steuersignale) zu generieren, um den Fortgang einer Katheterablation, einer Kardiostimulation, einer Herz-, Kreislauf- und/oder Lungenunterstützungseinrichtung, und/oder einer Neurostimulation zu beeinflussen bzw. zu kontrollieren. Fig. 1 zeigt ferner zwei Elektrokardiographiesignale S1 und S2 mit zwei hochfrequenten Elektrostimulationen.

**Fig. 2** zeigt schematisch eine beispielhafte Vorrichtung 200 zum transösophagealen Bioimpedanzmonitoring bei kardialer Resynchronisationstherapie oder zur Optimierung einer Herz-, Kreislauf- und Lungenunterstützungseinrichtung, insbesondere bei Hochfrequenz-, Kryo-, Ultraschall- oder Laserablation von Vorhofflimmern durch Isolation der Pulmonalvenen im linken Vorhof. Die Vorrichtung 200 ist ein Beispiel einer Vorrichtung zur transösophagealen kardiologischen Behandlung und/oder Diagnose.

Die Vorrichtung 200 umfasst eine Ösophaguselektrodensonde 10 mit mehreren in Reihen angeordneten Elektroden 12 zur Bioimpedanzmessung, wobei eine erste Reihe von Elektroden 12A auf der herznahen Seite 14 und eine Reihe von Elektroden 12B auf der herzabgewandten Seite 16 der Ösophaguselektrodensonde 10 angeordnet sind. Die Elektroden 12 stehen in Signalverbindung mit der Steuer- und Auswerteeinrichtung 30.

Die Vorrichtung zum transösophagealen Bioimpedanzmonitoring 200 und insbesondere die Steuer- und Auswerteeinrichtung 30 sind eingerichtet, Gewebeveränderungen kontinuierlich mit den dem Herzen zugewandten Elektroden 12 zu überwachen, die Bioimpedanzmessung der dem Herzen zugewandten Elektroden 12A mit der Bioimpedanzmessung der herzabgewandten Elektroden 12B zu vergleichen und Informationen daraus abzuleiten und/oder anzuzeigen. Gleichzeitig kann ein transösophageales hämodynamisches Monitoring durchgeführt werden.

Fig. 2 zeigt ein beispielhaftes transösophageales Impedanzkardiographiesignal S3 einer biventrikularen Stimulation. Dies kann zum Beispiel bei einer Pulmonalvenenisolation zur Behandlung von Vorhofflimmern bei einem implantierten kardialen Resynchronisationstherapie (CRT) System verwendet werden. Insbesondere zeigt Fig. 2 den rechtsatrialen (RAP) und rechtsventrikulären (RVP) Stimulationsimpuls bei 200ms atrioventrikulärem Delay des implantierten CRT Systems und die linksatrialen (LA) und linksventrikulären EKG Signale bei optimaler biventrikulärer Stimulation mit LA Signal zeitlich vor dem RVP Signal.

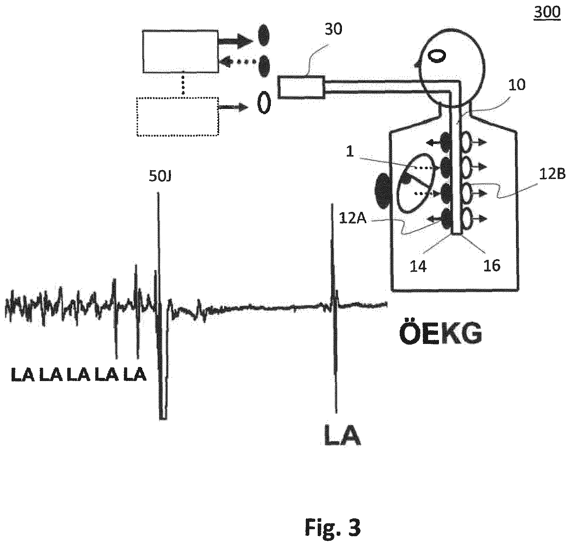

**Fig. 3** zeigt schematisch eine beispielhafte Vorrichtung zur transösophagealen Neurostimulation und Kardiostimulation 300, insbesondere zur transösophagealen linksatrialen und/oder linksventrikulären Stimulation und Elektrokardiographie im Rahmen der Diagnostik und Therapie bradykarder und tachykarder Herzrhythmusstörungen, z. B. Initiierung und Terminierung von AV-Knoten-Reentry-Tachykardien (AVNRT), AV-Reentry-Tachykardien (AVRT) und Vorhofflattern, oder zur transösophagealen linksventrikulären Stimulation im Rahmen einer temporären kardialen Resynchronisationstherapie. Die Vorrichtung 300 ist ein Beispiel einer Vorrichtung zur transösophagealen kardiologischen Behandlung und/oder Diagnose.

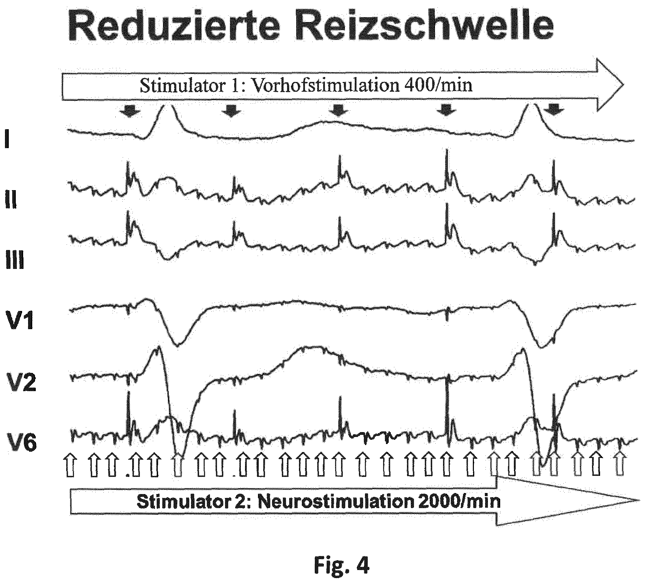

Die Vorrichtung 300 umfasst eine Ösophaguselektrodensonde 10 mit mehreren Elektroden 12 zur Neurostimulation auf der vom Herzen 1 abgewandten Seite 16 Ösophaguselektrodensonde 10 und mit mehreren Elektroden auf der dem Herzen zugewandten Seite 14 der Ösophaguselektrodensonde 10 zur transösophagealen elektrischen Kardioversion durch DC Energieabgabe, zum Beispiel von 50 J, bei Vorhofflattern und Vorhofflimmern. Die Ösophaguselektrodensonde 10 kann ebenfalls zusätzliche Elektroden umfassen, wie z.B. EKG Elektroden, die auf der dem Herz 1 zugewandten Seite 14 der Ösophaguselektrodensonde 10 angeordnet sind. Die Elektroden 12 stehen in Signalverbindung mit der Steuer- und Auswerteeinrichtung 30. An den Elektroden zur Neurostimulation werden elektrische Signale mit einer Frequenz von 100 bpm bis 3000 bpm, vorzugsweise von 1500 bpm bis 2000 bpm und einer Stärke von 5V bis 100 V für eine Dauer von 3 bis 30 Sekunden angelegt. Die transösophageale Neurostimulation ermöglicht eine Schmerzreduktion der transösophagealen kardialen Stimulation, wie in **Fig. 4** gezeigt. Insbesondere zeigt Fig. 4a transthorakale EKG Ableitungen I, II, III, V1, V2 und V6 bei hochfrequenter linksatrialer Stimulation mit 400 bpm mit Stimulator 1 und hochfrequenter nichtkardialer Stimulation mit 2000 bpm mit Stimulator 2.

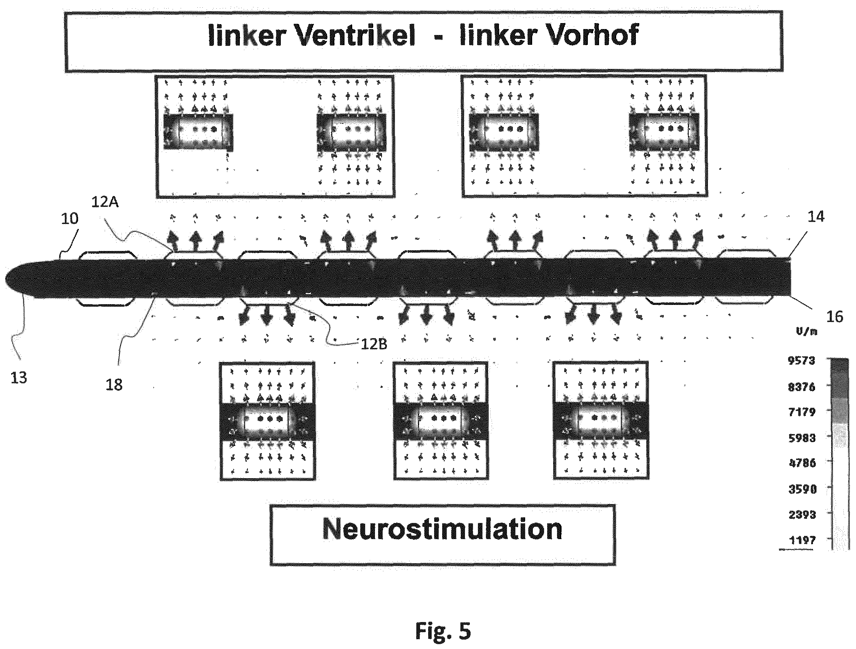

**Fig. 5** zeigt eine beispielhafte Ösophaguselektrodensonde 10 zur temporären transösophagealen bipolaren linksatrialen und linksventrikulären Stimulation und nichtkardialen Neurostimulation. Die Ösophaguselektrodensonde 10 umfasst einen länglichen, im Wesentlichen zylinderförmigen, flexiblen Sondenkörper 18 mit einem distalen Ende 13 und einem proximalen Ende. Die Längsachse des Sondenkörpers 18 fällt im Wesentlichen mit der Einführrichtung der Sonde zusammen. An dem Sondenkörper 18 sind mehrere Elektroden 12 in Reihen zwischen dem distalen und proximalen Ende angeordnet. Jede Elektrodenreihe erstreckt sich im Wesentlichen in Längsrichtung der Sonde 10. Die Elektroden 12B auf der vom Herzen abgewandten Seite der Ösophaguselektrodensonde 10 umfassen Elektroden zur Neurostimulation. Die Elektroden 12A auf der dem Herzen zugewandten Seite der Ösophaguselektrodensonde 10 umfassen Elektroden 12A zur bipolaren linksatrialen und bipolaren linksventrikulären Stimulation.

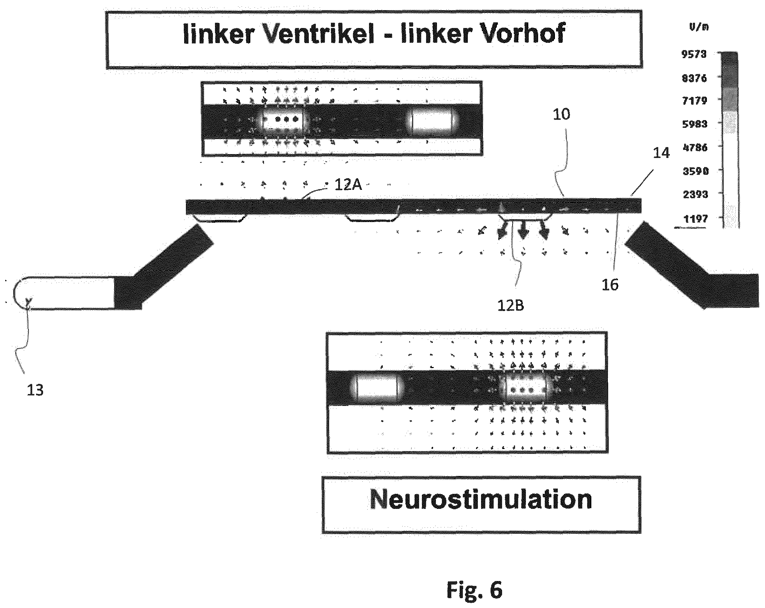

**Fig. 6** zeigt eine andere beispielhafte Ösophaguselektrodensonde 10 mit flachen Elektroden 12A zur Linksherzstimulation und Elektroden 12B zur Neurostimulation mit großem Elektroden-Rückenmark-Abstand auf der vom Herzen abgewandten Seite der Ösophaguselektrodensonde 10 und einer Vorrichtung zur Veränderung des Elektroden-Myokard-Abstandes, z.B. mit einem entsprechend vorgebogenem Mandrin/Stylet oder mit Form-Gedächtnis-Material.

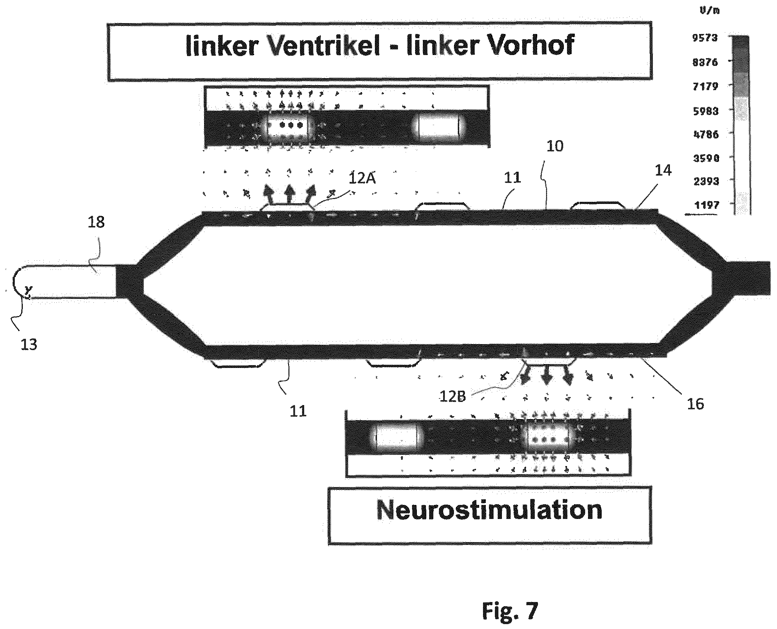

**Fig. 7** zeigt eine beispielhafte Ösophaguselektrodensonde 10 zur Linksherzstimulation und Neurostimulation mit mehreren länglichen Segmenten 11 mit Elektroden 12, die den Elektroden-Myokard-Abstand reduzieren können. Die länglichen Segmente 11 sind ähnlich wie die Segmente eines aufspannbaren Regenschirms angeordnet. Die Elektroden 12B auf der vom Herzen abgewandten Seite umfassen Elektroden zur Neurostimulation und Bioimpedanzmessung. Die Elektroden auf der dem Herzen zugewandten Seite umfassen Elektroden zur Linksherzstimulation, EKG und Bioimpedanzmessung. Die Anordnung der Elektroden 12 ist ähnlich der in Fig. 5 gezeigten Ösophaguselektrodensonde 10. Ebenfalls umfasst die Ösophaguselektrodensonde 10 eine Vorrichtung zur Veränderung des Elektroden-Myokard-Abstandes und des Elektroden-Rückenmark-Abstandes, z.B. mit einem entsprechend vorgebogenem Mandrin/Stylet oder mit Form-Gedächtnis-Material.

Die Elektroden 12 weisen eine im Wesentlichen halbsphärische oder halbzylindrische Form mit einer im Wesentlichen planen Fläche und einer leitfähigen gewölbten Fläche auf. Insbesondere sind auf der vom Herzen abgewandten Seite 16 Elektroden 12B zur Neurosimulation und auf der dem Herz zugewandten Seite Elektroden 12A zur Kardiostimulation angebracht.

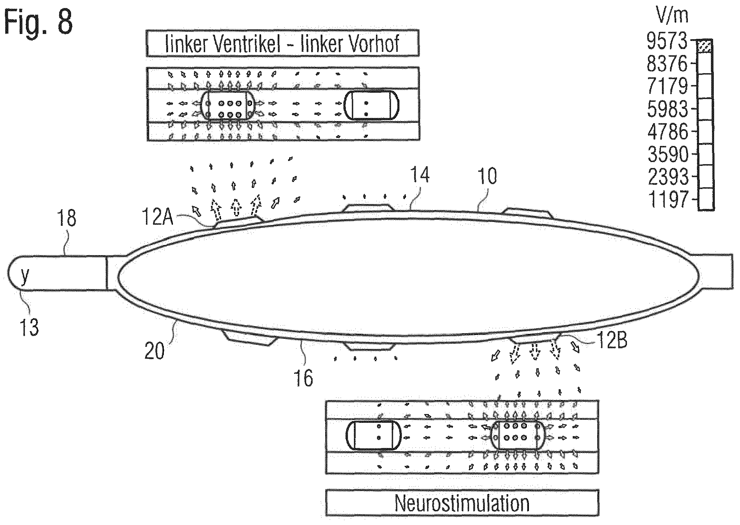

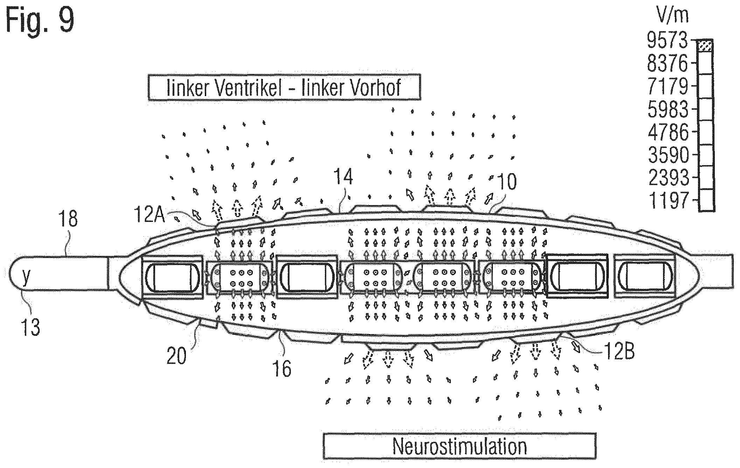

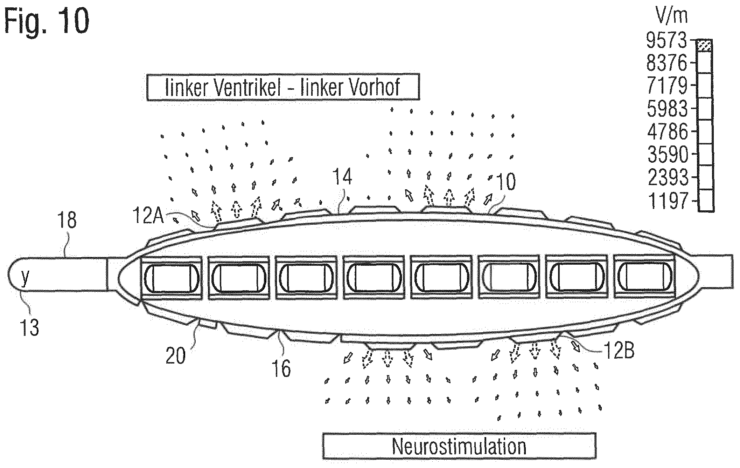

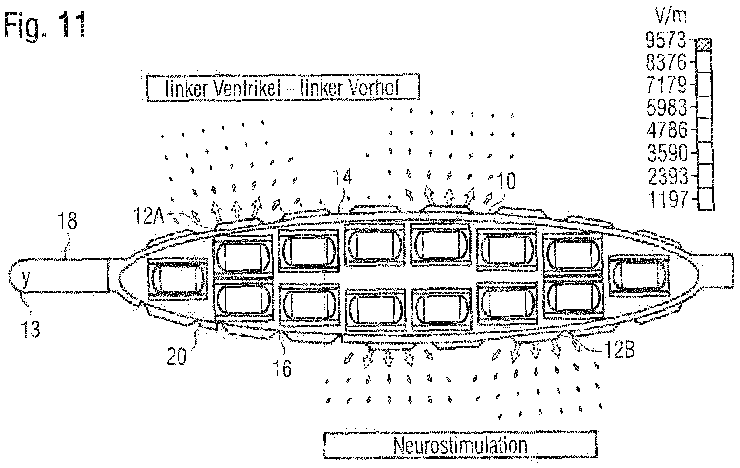

**Fig. 8** bis **11** zeigen jeweils andere beispielhafte Ösophaguselektrodensonden 10 zur temporären transösophagealen linksatrialen und/oder linksventrikulären Stimulation, und/oder zur Elektrokardiographie, und/oder zum hämodynamischen Monitoring mit reduziertem Elektroden-Myokard-Abstand und/oder zur temporären transösophagealen Neurostimulation mit reduziertem Elektroden-Rückenmark-Abstand. Die Ösophaguselektrodensonden 10 weisen einen aufblasbaren Katheterballon 20 auf, auf dem Elektroden 12 angebracht sind.

Der aus einem biokompatiblen elastischen Material ausgebildete Katheterballon 20 ist an dem zylinderförmigen Sondenkörper 18 angebracht. Beispielsweise kann der Katheterballon 10 an dem distalen und proximalen Ende des Sondenkörpers 18 befestigt sein. Wenn sich die Ösophaguselektrodensonde 10 an ihrer korrekten Position in der Speiseröhre des Patienten befindet, wird der Katheterballon 20 aufgeblasen, so dass dieser in engen Kontakt mit der Speiseröhre des Patienten kommt. Figuren 8 bis 11 zeigen jeweils die Ösophaguselektrodensonde 10 mit dem aufgeblasenen Katheterballon 20. In diesem Zustand tritt die leitfähige Elektrodenfläche der Elektroden 12 in niederohmigen Kontakt mit der Speiseröhre des Patienten, so dass die Stimulationen und/oder Messungen durchgeführt werden können.

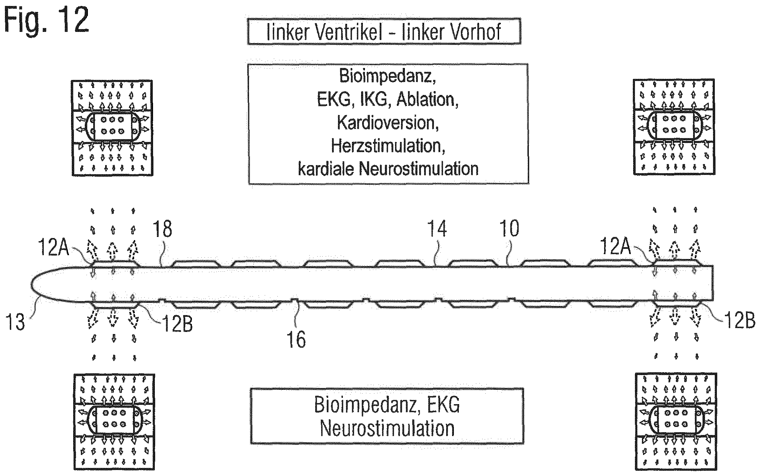

**Fig. 12** zeigt eine beispielhafte Ösophaguselektrodensonde 10 zur Bioimpedanzmessung und optional zu weiteren Messungen und/oder Behandlungen, wie z.B. zur EKG, IKG, Katheterablation, Kardioversion, Herzstimulation, kardiale Neurostimulation, insbesondere zur gerichteten Linksherzstimulation ohne Möglichkeit einer Reduktion des Elektroden-Myokard-Abstandes. Die Neurostimulation kann wahlweise als kardiale Neurostimulation in Herzrichtung und/oder nichtkardialer Neurostimulation in Richtung Wirbelsäule erfolgen.

Die Ösophaguselektrodensonde 10 weist mehrere, in Reihen in Längsrichtung des Sondenkörpers 18 angeordnete Elektroden 12 auf. Die Elektroden 12A auf der dem Herzen zugewandten Seite 14 der Ösophaguselektrodensonde 10 umfassen Elektroden zur Bioimpedanzmessung sowie optional Elektroden zur IKG, EKG, Kardioversion, Katheterablation; Neurostimulation und/oder Herzstimulation. Die Elektroden 12B auf der vom Herzen abgewandten Seite 16 der Ösophaguselektrodensonde 10 umfassen Elektroden zur Bioimpedanzmessung sowie optional auch Elektroden zur EKG und Neurostimulation. Die proximalen Elektroden 12A sind Elektroden zur unipolaren oder bipolaren linksventrikulären Stimulation und Elektrokardiographie und Bioimpedanz und die proximalen Elektroden 12A sind Elektroden zur unipolaren oder bipolaren linksatrialen Stimulation und Elektrokardiographie und Bioimpedanz ohne Katheterballon.

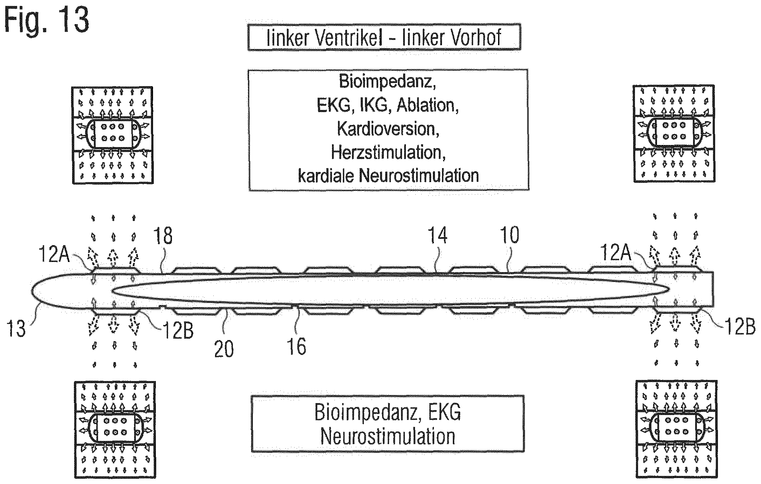

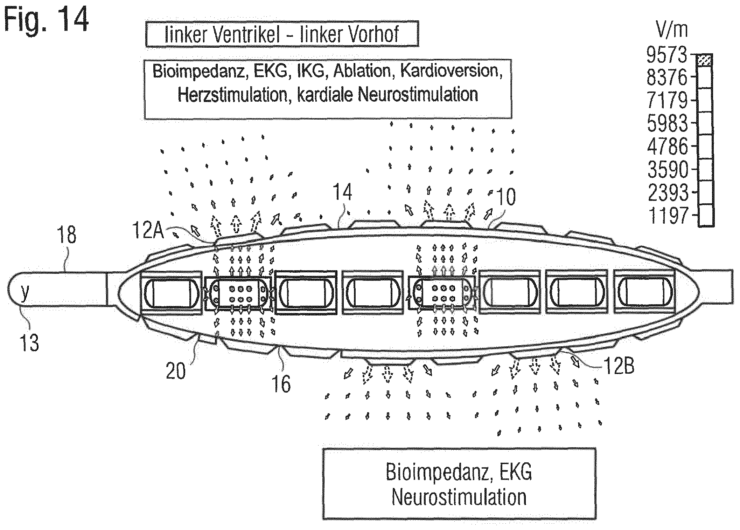

**Figuren 13** und **14** zeigen jeweils andere beispielhafte Ösophaguselektrodensonden 10 zur Biompedanzmessung und optional zu weiteren Messungen und/oder Behandlungen, wie z.B. zur Linksherzstimulation, Linksherz-Elektrokardiographie, Linksherz-Bioimpedanz, und/oder Neurostimulation. Die Neurostimulation kann wahlweise als kardiale Neurostimulation in Herzrichtung und/oder nichtkardialer Neurostimulation in Richtung Wirbelsäule erfolgen.

**Fig. 13** zeigt eine beispielhafte Ösophaguselektrodensonde 10 mit einem nicht aufgeblasenen Katheterbalon 20. **Fig. 14** zeigt eine beispielhafte Ösophaguselektrodensonde mit einem aufgeblasenen Katheterballon 20.

Die Ösophaguselektrodensonde 10 weist vier symmetrisch angeordnete Elektrodenreihen zur Stimulation, EKG, Bioimpedanz, kardiale Neurostimulation, Katheterablation, etc. auf. Der Unterschied zu den bisherigen Sonden ist, dass die bipolare Stimulation und/oder Elektrokardiographie/Impedanz zwischen zwei benachbarten Elektroden in benachbarten Elektrodenreihen realisiert werden kann. Damit können zum Beispiel lokalere EKGs erfasst werden und das linke Herz lokaler stimuliert werden.

Zwei oder mehrere der Elektroden 12 können wie oben beschrieben zusammen geschaltet und/oder angesteuert werden.

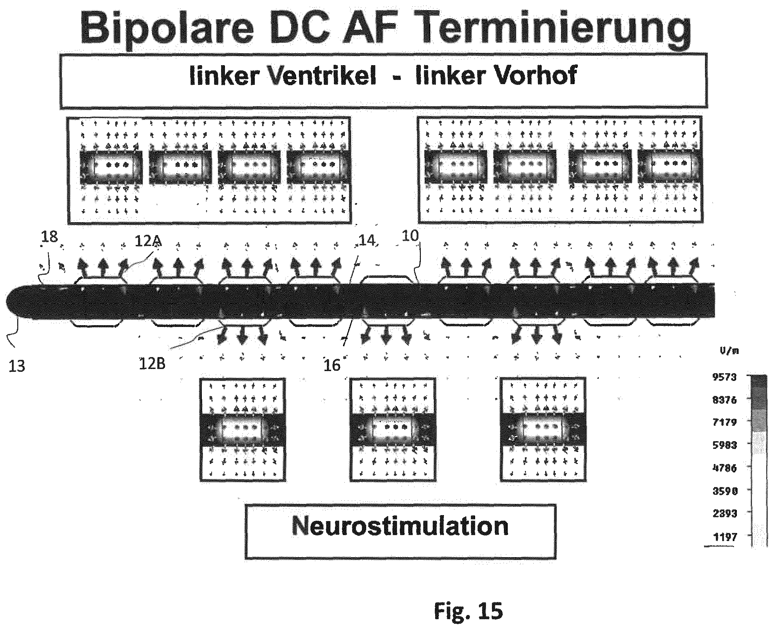

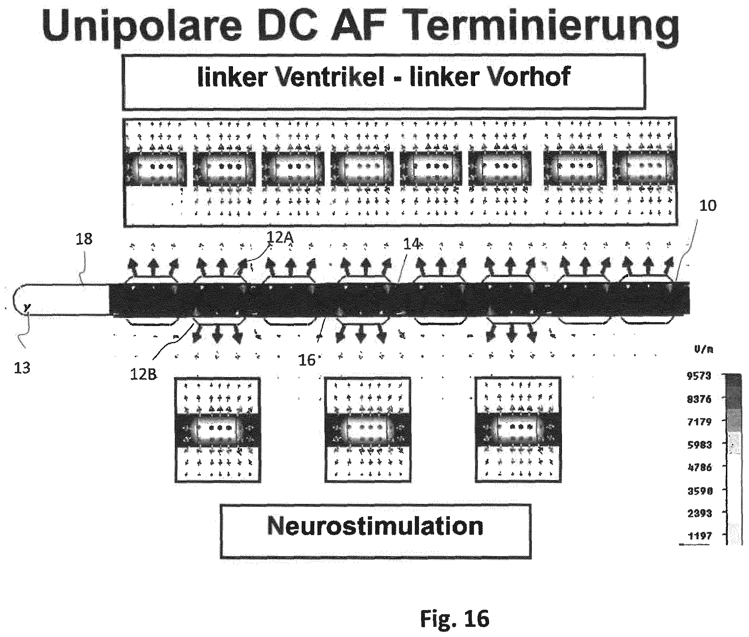

**Fig. 15** zeigt eine beispielhafte Ösophaguselektrodensonde 10 zur bipolaren DC Kardioversion zur Terminierung von Vorhofflimmern, Vorhofflattern und Kombination mit Stimulation, EKG und Impedanz. **Fig. 16** zeigt eine beispielhafte Ösophaguselektrodensonde 10 zur unipolaren DC Kardioversion zur Terminierung von Vorhofflimmern, Vorhofflattern und Kombination mit Stimulation, EKG und Impedanz.

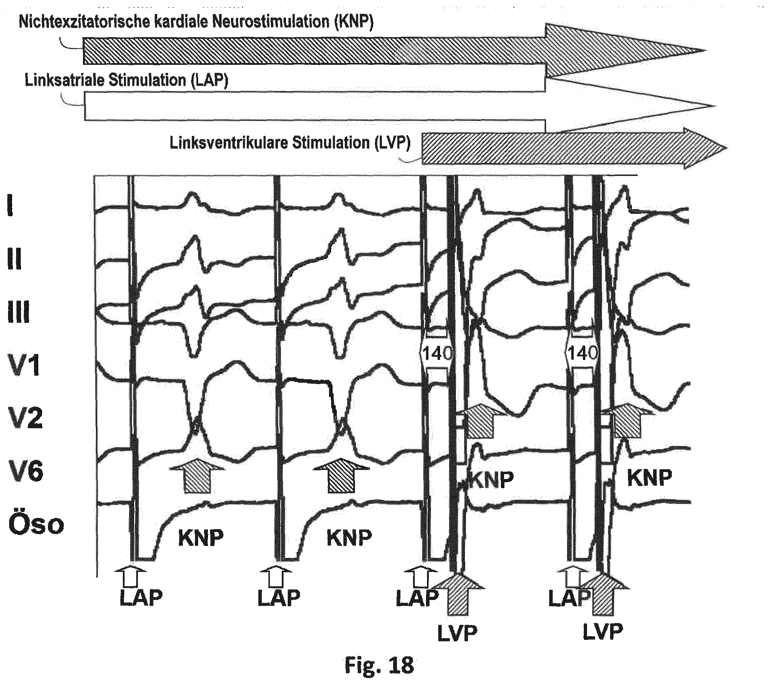

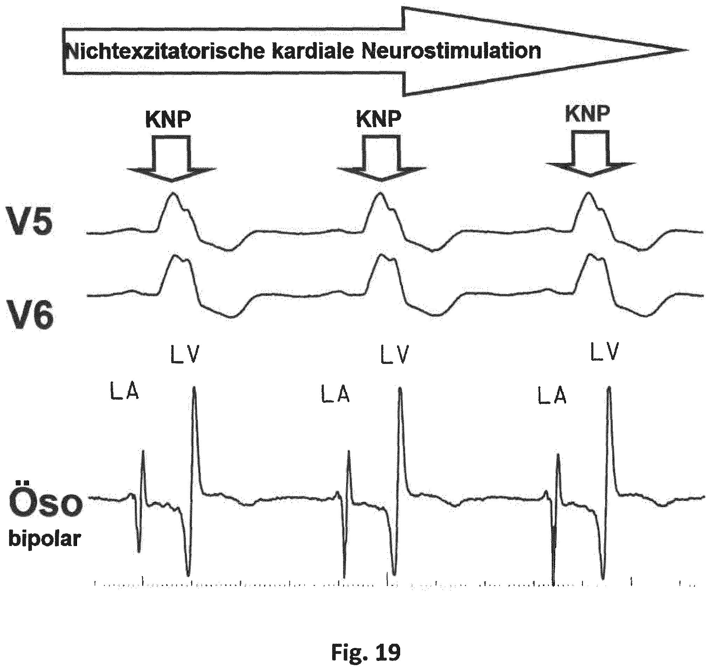

**Figuren 18** und **19** zeigen jeweils Beispiele für kardiale Neurostimulationen, welche mit Hilfe der oben beschriebenen Ösophaguselektrodensonden 10 durchgeführt werden können. Insbesondere sind in den Figuren 18 und 19 die transthorakalen EKGs mit den Ableitungen I, II, III, V1, V2, V5 und V6 dargestellt. **Fig. 18** zeigt eine beispielhafte kardiale Neurostimulation bei linksatrialer und linksventrikulärer Stimulation. **Fig. 19** zeigt eine beispielhafte kardiale Neurostimulation und transthorakale und transösophageale Elektrokardiographie bei Sinusrhythmus und Schenkelblockierung. Die kardiale Neurostimulation kann eine nichtexzitatorischen kardiale Neurostimulation (KNP) sein, d.h. eine hochfrequente gerichtete Stimulation in der absoluten ventrikulären Refraktärzeit, wobei die Stimulation innerhalb des ORS-Komplexes abgegeben wird. Die Signale zur Steuerung und Regelung realisieren die Impulsabgabe im ORS-Komplex und verhindern eine Impulsabgabe außerhalb des QRS-Komplexes.

Die kardiologischen Behandlungen und/oder Messungen mit den Ösophaguselektrodensonden 10 gemäß unterschiedlichen Aspekten der Erfindung können mit Hilfe eines digitalen Herzmodells simuliert werden, das z.B. auf 3D-CAD-Technologie basiert. Nachfolgend wird ein anatomisch korrektes 3D CAD Herzrhythmusmodell (HRM) zur Simulation von elektrophysiologischen Untersuchungen (EPU) und Hochfrequenz (HF) Ablationen beschrieben. Mit diesem Modell lassen sich komplexe kardiale rhythmologische Strukturen, intrakardiale und ösophageale Elektrodenkatether und Herzschrittmacherelektroden elektrisch und thermisch simulieren. Dies ist für die individualisierte Optimierung der Katheter und des Katheterablationsvorgangs sowie von Herzrhythmusimplantaten und für die Optimierung langatmiger und kostspieliger klinischer Studien von großer Bedeutung. Gleichermaßen wird das Risiko der Patientengefährdung auf ein Minimum reduziert und kann im Rahmen der Lehre und Forschung auf dem Gebiet der Diagnostik und Therapie von Herzrhythmusstörrungen eingesetzt werden. Mit Hilfe des vorgeschlagenen 3D Herzrhythmusmodells können Ösophaguselektrodensonden patientenoptimiert und individualisiert mit Hilfe der 3D Drucktechnik als Prototyp oder Serienprodukt hergestellt werden.

Das vorgeschlagene 3D Herzrhythmusmodell umfasst Myokard, Herzklammern, Erregungsbildung, Reizleitung, Ösophagus und intrakardiale Elektrodenkatheter (als ein Beispiel einer Ösophaguselektrodensonde) für die Simulation von elektrophysiologischen Untersuchungen (EPU), Hochfrequenz- (HF) Ablation, Herzschrittmachertherapie und unterschiedlichen bradykarden und tachykarden Herzrhythmen. Insbesondere werden im 3D Herzrhythmusmodell Sinusknoten, Bachmann Bündel, AV Knoten, His Bündel und rechts- und linksventrikuläre Tawaraschenkel modelliert. Die Anatomie kann zum Beispiel auf Basis von MRT Aufnahmen und anatomischen Schnittbildern maßstabsgetreu modelliert werden. Ebenfalls können verschiedene Elektrodenkatheter und insbesondere Ösophgauselektrodensonden modelliert und an geeigneten Stellen im Herzmodell positioniert werden. Die für die Herzkatheter verwendeten Materialen und/oder die Gewebeparameter der Herzanatomie und Rhythmologie können aus einer Datenbank ausgelesen werden, wobei die Datenbank Bestandteil der Simulationssoftware sein kann.

Das vorgeschlagene Herzrhythmusmodell kann beispielsweise auf CST STUDIO SUITE^{®} basieren, einer Simulationssoftware der CST Computer Simulation Technology AG, Darmstadt, mit der eine Vielzahl von elektromagnetischen Simulationen durchgeführt werden kann. Ein weiterer Vorteil ist es, dass eine große Anzahl unterschiedlicher Materialparameter zur Verfügung stehen. So beinhaltet die Material Library von CST eine Vielzahl von Materialien, die sich auf menschliches Körpergewebe beziehen, wobei in diesen Werkstoffen die notwendigen Parameter, wie elektrische Leitfähigkeit oder Wärmekapazität, enthalten sind. Selbstverständlich kann auch andere Simulationssoftware verwendet werden.

Zur Realisierung des Herzmodells werden die vier Herzkammern und das Erregungsbildungs- und Reizleitungssystem des Herzens modelliert, wobei Materialparameter (wie elektrische Leitfähigkeit, Wärmekapazität, etc.) verwendet werden, die auf das menschliche Körpergewebe bezogen sind. Vorzugsweise wird im Herzmodell die Gewebekühlung durch einen eingerechneten Blutfluss und Metabolismus berücksichtigt. Ferner können ebenfalls Impedanzänderungen des Gewebes berücksichtigt werden.

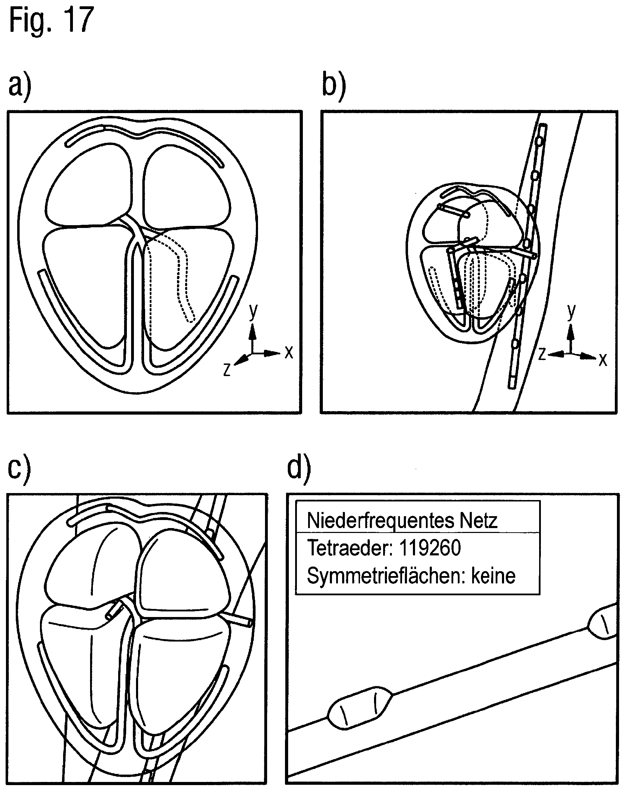

**Fig. 17** zeigt ein beispielhaftes 3D-CAD Herzmodell, das ein tetrahedrales Netz verwendet, wobei Fig. 17a das 3D-CAD Herzrhythmusmodell mit Erregungsleitung, Fig. 17b das Herzmodell mit positionierten Herzkathetern, Fig. 17c das tetrahedrale Netz der Herzkammern und der Erregungsleitung und Fig. 17d einen Ausschnitt des tetrahedralen Netzes des Ösophaguskatheters bzw. der Ösophaguselektrodensonde zeigen.

Das Herzmodell weist insbesondere die folgenden Merkmale auf:
- 3D-Modellierung der Organe durch eine Splinefunktion, wie z.B. ein tetrahedrales Netz bzw. Mesh;
- Komplette Herzleitung (Sinusknoten, AVKnoten, Tawara Schenkel, Bachmann Bündel) durch Splinefunktionen;

- Positionierung der Herzleitung anhand gemittelter MRT Daten;
- Realisierung der elektrischen Leitfähigkeit durch definierte Spannungspfade entlang der Herzleitung;
- Modellierung des Ösophagus durch Splinefunktionen anhand MRT Daten;
- Modellierung von unterschiedlichen Kathetern, wie zum Beispiel multipolaren Elektrodenkathetern;
- Modellierung von unterschiedlichen bradykarden und tachykarden Herzrhythmusstörungen;
- Modellierung von Elektrostimulation, Neurostimulation und/oder Elektrokardiographie;
- Modellierung von unterschiedlichen Ablationen bzw. Katheterablationen, wie z.B. HF Ablationen;
- Erstellen eines Sinusknotensignals anhand EPU Daten durch Überlagern eines definierten Trapezoidal-Signals und eines Aktionspotentials;
- Definierung der elektrischen Herzerregung entlang der Herzleitung;
- Modellierung einer realistischen Herzaktion durch Festlegung der zeitlichen Abfolge der Signale und deren Amplitude;
- Definieren von Monitoringparametern mit getesteten Netzparametern und Auflösungseigenschaften der Monitoringfunktionen;
- Ableiten der Signale durch definierte 1D "Monitore" an den Kathetern;
- Umsetzung der Ablationstherapie durch das Überlagern eines Hochfrequentsignals und einer Energie;
- Schnittstellen und Datenformate für den 3D-Druck von patientenspezifischen Herzrhythmusmodellen mit und ohne Herzkatheter und/oder Ösophaguselektrodensonde und/oder elektromagnetische und/oder thermale Feldverläufen für medizinische Betreuung, Lehre und Forschung;
- Schnittstellen und Datenformate für den 3D-Druck von Ösophaguselektrodensonden als Prototypen und/oder nach Zulassung des 3D-Drucks als Serienprodukte.

Das Herzmodell ermöglicht insbesondere zeitliche Simulationen im Niederfrequenzbereich. Durch die Möglichkeit, materialunabhängig elektrische Potentiale anzulegen und Spannungspfade zu definieren, eignet sich das Herzmodell hervorragend für die Simulation von Erregungsleitungen innerhalb des Herzens und zur Simulation der elektrischen Herzstimulation und Elektrokardiographie mit intrakardialen und transösophagealen Elektrodenkathetern.

Das Herzmodell ermöglicht es ferner, ein Monitoring der elektrischen oder anderer Eigenschaften an definierten Punkten durchzuführen. Die Funktion des Monitorings an definierten Punkten ermöglicht die Ableitung simulierter Eigensignale des Herzens mit Hilfe unterschiedlicher Elektroden eines multipolaren Elektrodenkatheters. Die zeitliche Darstellung einer elektrischen Herzaktivität kann mittels LF Time Domain Solver als E-Feld visualisiert werden.

Ferner lassen sich unterschiedliche Anregungssignale innerhalb des Herzmodells erstellen, was die Rekonstruktion von unterschiedlichen Herzrhythmen ermöglicht. Ebenfalls kann eine thermale Simulation durchgeführt werden, wobei Wärme- und Leistungsquellen simuliert und je nach gewünschtem Ergebnis statisch oder im Zeitbereich über eine definierte Zeitspanne berechnet werden können. Durch die Simulation von Leistungsquellen im Zeitbereich konnte eine Therapie in Form einer HF Ablation durch die Möglichkeit der Definition eines hochfrequenten Sinussignals dargestellt werden. Mit dem 3D-Druck, Herzrhythmusmodellen und Elektrodenmodellen lassen sich patientenspezifische Herzrhythmusmodelle mit und ohne Herzkatheter und/oder Ösophaguselektrodensonden und/oder elektromagnetische und/oder thermale Feldverläufen für die medizinische Betreuung, Lehre und Forschung herstellen.

### Bezugszeichenliste

1 Herz
10 Ösophaguselektrodensonde
11 Längliche Segmente
12 Elektroden
12A Elektroden auf der dem Herzen zugewandten Seite der Ösophaguselektrodensonde
12B Elektroden auf der vom Herzen abgewandten Seite der Ösophaguselektrodensonde
13 distales Ende der Ösophaguselektrodensonde
14 dem Herzen zugewandte Seite der Ösophaguselektrodensonde
16 dem Herzen abgewandte Seite der Ösophaguselektrodensonde
18 Sondenkörper
20 Katheterballon
30 Steuer- und/oder Auswerteinrichtung
100 Vorrichtung zum transösophagealen Bioimpedanzmonitoring und zur Neurostimulation
200 Vorrichtung zum transösophagealen Bioimpedanzmonitoring
300 Vorrichtung zur transösophagealen Neurostimulation und Kardiostimulation;
S1 bis S3 Signale

## Patentansprüche

1. Ösophaguselektrodensonde (10), umfassend:
eine Bioimpedanzmesscinrichtung zur Messung der Bioimpedanz von zumindest einem Teil des die Ösophaguselektrodensonde (10) umgebenden Gewebes, wobei die Bioimpedanzmesseinrichtung mindestens eine erste Elektrode (12A) und mindestens eine zweite Elektrode (12B) umfasst, wobei die mindestens eine erste Elektrode (12A) auf einer im Gebrauch dem Herzen (1) zugewandten Seite (14) der Ösophaguselektrodensonde (10) angeordnet ist, und die mindestens eine zweite Elektrode (12B) auf einer im Gebrauch vom Herzen (1) abgewandten Seite der Ösophaguselektrodensonde (10) angeordnet ist, und
ferner umfassend
eine Neurostimulationeinrichtung, wobei die Neurostimulationeinrichtung mindestens eine auf der im Gebrauch vom Herzen abgewandten Seite (16) der Ösophaguselektrodensonde (10) angeordnete Elektrode (12B) für eine transösophageale Neurostimulation des zumindest einen Teils des die Ösophaguselektrodensonde (10) umgebenden Gewebes umfasst, **gekennzeichnet dadurch, dass** die Elektrode (12B) eingerichtet ist, transösophageale Neurostimulation mittels elektrischer Pulse mit einer Frequenz von 100 bpm bis 3000 bpm und einer Stärke von 5 V bis 100 V und einer Dauer von 3 Sekunden bis 10 Minuten durchzuführen.

2. Ösophaguselektrodensonde (10) nach Anspruch 1, ferner umfassend einen zylinderförmigen Sondenkörper (18) und einen aufblasbaren Katheterballon (20), welcher an den Sondenkörper (18) angebracht ist, wobei die Elektroden (12) der Bioimpedanzmesseinrichtung und/oder der Neurostimulationeinrichtung auf dem Katheterballon (20) angeordnet sind.

3. Ösophaguselektrodensonde (10) nach einem der vorangegangenen Ansprüche, umfassend ferner mindestens eine Einrichtung aus der Gruppe, bestehend aus:
einer Stimulationseinrichtung mit mindestens einer Elektrode zur transösophagealen kardialen Stimulation:
einer Elektrographieeinrichtung mit mindestens einer Elektrode zur Elektrographiemessung;
einer Echokardiographieeinrichtung mit mindestens einem Ultraschallsensor zur Echokardiographiemessung;
einer Temperaturmesseinrichtung mit mindestens einem Temperatursensor; und
einer pH-Wert Messeinrichtung mit mindestens einem pH-Wert Sensor zur pH-Wert Messung.

4. Ösophaguselektrodensonde (10) nach einem der vorangegangenen Ansprüche, wobei die Ösophaguselektrodensonde (10) eine Elektrographieeinrichtung mit mindestens einer ersten und mindestens einer zweiten Elektrode zur Elektrographiemessung umfasst,
wobei die mindestens eine erste Elektrode zur Elektrographiemessung auf einer im Gebrauch dem Herzen (1) zugewandten Seite (14) der Ösophaguselektrodensonde (10) angeordnet ist, und die mindestens eine zweite Elektrode zur Elektrographiemessung auf einer im Gebrauch vom Herzen (1) abgewandten Seite der Ösophaguselektrodensonde (10) angeordnet ist.

5. Vorrichtung (100, 200, 300) zur transösophagealen kardiologischen Behandlung oder zur transösophagealen kardiologischen Diagnose (100, 200, 300), umfassend:
eine Ösophaguselektrodensonde (10) nach einem der vorangegangenen Ansprüche; und
eine Steuer- und/oder Auswerteinrichtung, welche in Signalverbindung mit der Bioimpedanzmesseinrichtung steht, wobei die Steuer- und/oder Auswerteinrichtung (30) eingerichtet ist, ein erstes Bioimpedanzmesssignal von der mindestens einen ersten Elektrode und ein zweites Bioimpedanzmesssignal von der mindestens einen zweiten Elektrode der Bioimpedanzmesseinrichtung zu empfangen und zu vergleichen, und ein Kontrollsignal auf Basis des Vergleichs zu generieren.

6. Vorrichtung (100, 200, 300) nach Anspruch 5, wobei die Ösophaguselektrodensonde (10) eine Ösophaguselektrodensonde (10) nach Anspruch 5 ist, und wobei
die Steuer- und/oder Auswerteinrichtung (30) eingerichtet ist, ein erstes Elektrokardiographiemesssignal von der mindestens einen ersten Elektrode und ein zweites Elektrokardiographiemesssignal von der mindestens einen zweiten Elektrode der Elektrographieeinrichtung zu empfangen und zu vergleichen, und das Kontrollsignal auf Basis des Vergleichs zu generieren.

7. Vorrichtung (100, 200, 300) nach Anspruch 5 oder 6, ferner umfassend mindestens eine Einrichtung ausgewählt aus der Gruppe, bestehend aus
einer Ablationseinrichtung zum Durchführen einer Herzkatheterablation, wobei die Ablationseinrichtung in Signalverbindung mit der Steuer- und/oder Auswerteinrichtung (30) steht; und
einer Herz-, Kreislauf- und/oder Lungenunterstützungseinrichtung zur herzsynchronen Herz-, Kreislauf- und/oder Lungenunterstützung, wobei die Herz-, Kreislauf- und/oder Lungenunterstützungseinrichtung in Signalverbindung mit der Steuer- und/oder Auswerteinrichtung (30) steht.

8. Vorrichtung (100, 200, 300) nach Anspruch 7, wobei das Kontrollsignal mindestens ein Signal ist ausgewählt aus der Gruppe, bestehend aus:
einem Statussignal ist, das den Status oder den Wert zumindest eines Parameters der Herzkatheterablation oder der Herz-, Kreislauf- und/oder Lungenunterstützung anzeigt;
einem Warnsignal ist, das anzeigt, dass sich zumindest ein Parameter einer Herzkatheterablation oder der Herz-, Kreislauf- oder Lungenunterstützung außerhalb eines zulässigen Wertebereichs befindet oder größer oder kleiner als ein vorbestimmter Schwellenwert ist; und
einem Steuersignal zum Steuern oder Regeln einer Ablationseinrichtung und/oder einer Herz-, Kreislauf- und/oder Lungenunterstützungseinrichtung ist.

9. Vorrichtung (100, 200, 300) nach einem der Ansprüche 5 bis 8, wobei das Kontrollsignal ein Signal zum Beenden der Herzkatheterablation oder der Herz-, Kreislauf- und/oder Lungenunterstützung ist, und wobei das Kontrollsignal generiert wird, wenn die Differenz zwischen dem ersten Bioimpedanzmesssignal und dem zweiten Bioimpedanzmesssignal gleich oder größer als ein vorbestimmter Schwellenwert ist.

10. Vorrichtung (100, 200, 300) nach einem der Ansprüche 5 bis 9, ferner umfassend
eine Anzeigeeinrichtung, die in Signalverbindung mit der Ösophaguselektrodensonde (10) steht, wobei die Anzeigeeinrichtung eingerichtet ist, mindestens eines der Messsignale aus der Gruppe, bestehend aus dem ersten Bioimpedanzmesssignal, dem zweiten Bioimpedanzmesssignal und dem Kontrollsignal, anzuzeigen.

## Claims

1. An oesophageal electrode probe (10) comprising:
a bioimpedance measuring device for measuring the bioimpedance of at least part of the tissue surrounding the oesophageal electrode probe (10), the bioimpedance measuring device comprising at least one first electrode (12A) and at least one second electrode (12B), wherein the at least one first electrode (12A) is arranged, during use, on a side (14) of the esophageal electrode probe (10) facing the heart (1), and the at least one second electrode (12B) is arranged, during use, on a side of the esophageal electrode probe (10) facing away from the heart (1), and
further comprising
a neurostimulation device, said neurostimulation device comprising at least one electrode (12B), arranged, during use, on the side of the esophageal electrode probe (10) facing away from the heart, for transesophageal neurostimulation of at least part of the tissue surrounding the oesophageal electrode probe (10), **characterized in that** the electrode (12B) is configured to perform transesophageal neurostimulation by means of electric pulses having a frequency of 100 bpm to 3000 bpm and a strength of 5 V to 100 V and a duration of 3 seconds to 10 minutes.

2. The oesophageal electrode probe (10) according to claim 1, further comprising
a cylindrical probe body (18) and an inflatable catheter balloon (20) attached to the probe body (18), wherein the electrodes (12) of the bioimpedance measuring device and/or the neurostimulation device are arranged on the catheter balloon (20).

3. The oesophageal electrode probe (10) according to any one of the preceding claims, further comprising at least one device selected from the group consisting of:
a stimulation device having at least one electrode for transoesophageal cardiac stimulation;
an electrography device having at least one electrode for electrography measurement;
an echocardiography device having at least one ultrasound sensor for echocardiography measurement;
a temperature measuring device having at least one temperature sensor; and
a pH measuring device with at least one pH sensor for pH measurement.

4. The oesophageal electrode probe (10) according to any one of the preceding claims, wherein the oesophageal electrode probe (10) comprises a electrography device having at least one first and at least one second electrode for electrography measurement,
wherein the at least one first electrode for electrography measurement is arranged, during use, on a side (14) of the oesophageal electrode probe (10) facing the heart (1), and the at least one second electrode for electrography measurement is arranged, during use, on a side of the oesophageal electrode probe (10) facing away from the heart (1).

5. A device (100, 200, 300) for transesophageal cardiological treatment or for transesophageal cardiological diagnosis (100, 200, 300), comprising:
an oesophageal electrode probe (10) according to any one of the preceding claims; and
a control and/or evaluation device which is in signal connection with the bioimpedance measuring device, wherein the control and/or evaluation device (30) is configured to receive and compare a first bioimpedance measuring signal from the at least one first electrode and a second bioimpedance measuring signal from the at least one second electrode of the bioimpedance measuring device, and to generate a control signal on the basis of the comparison.

6. The device (100, 200, 300) according to claim 5, wherein the esophageal electrode probe (10) is an esophageal electrode probe (10) according to claim 5, and wherein
the control and/or evaluation device (30) is arranged to receive and compare a first electrocardiography measurement signal from the at least one first electrode and a second electrocardiography measurement signal from the at least one second electrode of the electrography device, and to generate the control signal based on the comparison.

7. The device (100, 200, 300) according to claim 5 or 6, further comprising at least one device selected from the group consisting of
an ablation device for performing cardiac catheter ablation, the ablation device being in signal connection with the control and/or evaluation device (30); and
a cardiac, circulatory and/or pulmonary support device for heart-synchronous cardiac, circulatory and/or pulmonary support, wherein the cardiac, circulatory and/or pulmonary support device is in signal connection with the control and/or evaluation device (30).

8. The device (100, 200, 300) according to claim 7, wherein the control signal is at least one signal selected from the group consisting of:
a status signal indicating the status or value of at least one parameter of cardiac catheter ablation or of cardiac, circulatory and/or pulmonary support;
a warning signal indicating that at least one parameter of cardiac catheter ablation or cardiac, circulatory or pulmonary support is outside an acceptable range of values or is greater than or less than a predetermined threshold; and
a control signal for controlling or regulating an ablation device and/or a cardiac, circulatory and/or pulmonary support device.

9. The device (100, 200, 300) according to any one of claims 5 to 8, wherein the control signal is a signal to terminate cardiac catheter ablation or cardiac, circulatory and/or pulmonary support, and wherein the control signal is generated when the difference between the first bioimpedance measurement signal and the second bioimpedance measurement signal is equal to or greater than a predetermined threshold.

10. The device (100, 200, 300) according to any one of claims 5 to 9, further comprising
a display device in signal communication with the oesophageal electrode probe (10), the display device being configured to display at least one of the measurement signals selected from the group consisting of the first bioimpedance measurement signal, the second bioimpedance measurement signal and the control signal.

## Revendications

1. Sonde à électrode oesophagienne (10), comportant :
un dispositif de mesure de bioimpédance pour la mesure de la bioimpédance d'au moins une partie du tissu entourant la sonde à électrode oesophagienne (10), dans laquelle le dispositif de mesure de bioimpédance comporte au moins une première électrode (12A) et au moins une deuxième électrode (12B), dans laquelle l'au moins une première électrode (12A) est disposée sur une face (14) de la sonde à électrode oesophagienne (10) orientée vers le coeur (1) en cours d'utilisation, et l'au moins une deuxième électrode (12B) est disposée sur une face de la sonde à électrode oesophagienne (10) opposée au coeur(1) en cours d'utilisation, et comportant en outre
un dispositif de neurostimulation, dans laquelle le dispositif de neurostimulation comporte au moins une électrode (12B) disposée sur la face (16) de la sonde à électrode oesophagienne (10) opposée au coeur en cours d'utilisation pour une neurostimulation transoesophagienne de l'au moins une partie du tissu entourant la sonde à électrode oesophagienne (10), **caractérisée en ce que** l'électrode (12B) est conçue pour réaliser une neurostimulation transoesophagienne au moyen d'impulsions électriques avec une fréquence de 100 bpm à 3 000 bpm et une amplitude de 5 V à 100 V et une durée de 3 secondes à 10 minutes.

2. Sonde à électrode oesophagienne (10) selon la revendication 1, comportant en outre
un corps de sonde cylindrique (18) et un ballonnet de cathéter gonflable (20), lequel est monté sur le corps de sonde (18), dans laquelle les électrodes (12) du dispositif de mesure de bioimpédance et/ou du dispositif de neurostimulation sont disposées sur le ballonnet de cathéter. (20)

3. Sonde à électrode oesophagienne (10) selon l'une des revendications précédentes, comportant en outre au moins un dispositif parmi le groupe constitué :
d'un dispositif de stimulation avec au moins une électrode pour la stimulation cardiaque transoesophagienne ;
d'un dispositif d'électrographie avec au moins une électrode pour la mesure d'électrographie ;
d'un dispositif d'échocardiographie avec au moins un capteur à ultrasons pour la mesure d'échocardiographie ;
d'un dispositif de mesure de température avec au moins un capteur de température ; et
d'un dispositif de mesure de valeur de pH avec au moins un capteur de valeur de pH pour la mesure de valeur de pH.

4. Sonde à électrode oesophagienne (10) selon l'une des revendications précédentes, la sonde à électrode oesophagienne (10) comportant un dispositif d'électrographie avec au moins une première et au moins une deuxième électrode pour la mesure d'électrographie, dans laquelle l'au moins une première électrode pour la mesure d'électrographie est disposée sur une face (14) de la sonde à électrode oesophagienne (10) orientée vers le coeur (1) en cours d'utilisation, et l'au moins une deuxième électrode pour la mesure d'électrographie est disposée sur une face de la sonde à électrode oesophagienne (10) opposée au coeur (1) en cours d'utilisation.

5. Dispositif (100, 200, 300) pour le traitement cardiologique transœsophagien ou pour le diagnostic cardiologique transoesophagien (100, 200, 300), comportant :
une sonde à électrode oesophagienne (10) selon l'une des revendications précédentes ; et
un dispositif de commande et/ou d'évaluation, lequel est en communication de signal avec le dispositif .de mesure de b.ioimpédance, dans lequel le dispositif de commande et/ou d'évaluation (30) est conçu pour recevoir et pour comparer un premier signal de mesure de bioimpédance en provenance de l'au moins une première électrode et un deuxième signal de mesure de bioimpédance en provenance de l'au moins une deuxième électrode du dispositif de mesure de bioimpédance, et pour générer un signal de contrôle sur la base de la comparaison.

6. Dispositif (100, 200, 300) selon la revendication 5, dans lequel la sonde à électrode oesophagienne (10) est une sonde à électrode oesophagienne (10) selon la revendication 5, et dans lequel le dispositif de commande et/ou d'évaluation (30) est conçu pour recevoir et pour comparer un premier signal de mesure d'électrocardiographie en provenance de l'au moins une première électrode et un deuxième signal de mesure d'électrocardiographie en provenance de l'au moins une deuxième électrode du dispositif d'électrocardiographie, et pour générer le signal de contrôle sur la base de la comparaison.

7. Dispositif (100, 200, 300) selon la revendication 5 ou la revendication 6, comportant en outre au moins un dispositif sélectionné dans le groupe constitué
d'un dispositif d'ablation pour réaliser une ablation par cathéter cardiaque, dans lequel le dispositif d'ablation est en communication de signal avec le dispositif de commande et/ou d'évaluation (30) ; et
d'un dispositif d'assistance cardiaque, circulatoire et/ou pulmonaire pour l'assistance cardiaque, circulatoire et/ou pulmonaire synchrone avec le coeur, dans lequel le dispositif d'assistance cardiaque, circulatoire et/ou pulmonaire est en communication de signal avec le dispositif de commande et/ou d'évaluation (30).

8. Dispositif (100, 200, 300) selon la revendication 7, dans lequel le signal de contrôle est au moins un signal sélectionné dans le groupe constitué :
d'un .signal de.statut lequel indique le statut ou la valeur .d'au moins un paramètre de l'ablation par cathéter cardiaque ou de l'assistance cardiaque, circulatoire et/ou pulmonaire ;
d'un signal d'avertissement, lequel indique qu'au moins un paramètre d'une ablation par cathéter cardiaque ou de l'assistance cardiaque, circulatoire ou pulmonaire se trouve en dehors d'une plage de valeurs autorisée ou est supérieur ou inférieur à une valeur seuil prédéterminée ; et
d'un signal de commande pour la commande ou la régulation d'un dispositif d'ablation et/ou d'un dispositif d'assistance cardiaque, circulatoire et/ou pulmonaire.

9. Dispositif (100, 200, 300) selon l'une des revendications 5 à 8, dans lequel le signal de contrôle est un signal pour mettre fin à l'ablation par cathéter cardiaque ou à l'assistance cardiaque, circulatoire et/ou pulmonaire, et dans lequel le signal de contrôle est généré lorsque la différence entre le premier signal de mesure de bioimpédance et le deuxième signal de mesure de bioimpédance est égale ou supérieure à une valeur seuil prédéterminée.

10. Dispositif (100, 200, 300) selon l'une des revendications 5 à 9, comportant en outre
un dispositif d'affichage, lequel est en communication de signal avec la sonde à électrode oesophagienne (10), dans lequel le dispositif d'affichage est conçu pour afficher au moins l'un des signaux de mesure parmi le groupe constitué du premier signal de mesure de bioimpédance, du deuxième signal de mesure de bioimpédance et du signal de contrôle.
